# EUROPEAN PATENT SPECIFICATION

(11) **EP 4 179 320 B1**
(45) Date of publication and mention of the grant of the patent: **31.07.2024**
(21) Application number: 21742115.5
(22) Date of filing: 09.07.2021
(51) Int. Cl.: G01N 33/50, G01N 33/68

(54) **SYSTEM**
SYSTEM
SYSTÈME

(30) Priority: 10.07.2020 GB 202010620
(43) Date of publication of application: 17.05.2023
(73) Proprietor: Gtinvent Limited, Aberdeen Scotland AB24 5RP (GB)
(72) Inventor: POLLACK, Saskia, Julie, Brighton BN1 9QG (GB); MARSHALL, Karen, Elizabeth, Brighton BN1 9QG (GB); SERPELL, Louise, Charlotte, Brighton BN1 9QG (GB); WISCHIK, Claude, Michel, Aberdeen AB24 5RP (GB); HARRINGTON, Charles, Robert, Aberdeen Aberdeen AB25 2ZD (GB)
(74) Representative: Nick, Amy Sarah
(86) International application number: PCT/EP2021/069138
(87) International publication number: WO 2022/008712

(56) References cited:
- CHARLES R. HARRINGTON ET AL: "Cellular Models of Aggregation-dependent Template-directed Proteolysis to Characterize Tau Aggregation Inhibitors for Treatment of Alzheimer Disease", JOURNAL OF BIOLOGICAL CHEMISTRY, vol. 290, no. 17, 10 March 2015 (2015-03-10), US, pages 10862 - 10875, XP055727618, ISSN: 0021-9258, DOI: 10.1074/jbc.M114.616029
- AL-HILALY YOUSSRA K ET AL: "Alzheimer's Disease-like Paired Helical Filament Assembly from Truncated Tau Protein Is Independent of Disulfide Crosslinking", JOURNAL OF MOLECULAR BIOLOGY, vol. 429, no. 23, 15 September 2017 (2017-09-15), pages 3650 - 3665, XP085262223, ISSN: 0022-2836, DOI: 10.1016/J.JMB.2017.09.007
- AL-HILALY YOUSSRA K. ET AL: "Tau (297-391) forms filaments that structurally mimic the core of paired helical filaments in Alzheimer's disease brain", FEBS LETTERS, vol. 594, no. 5, 1 December 2019 (2019-12-01), NL, pages 944 - 950, XP055848181, ISSN: 0014-5793, Retrieved from the Internet <URL:https://onlinelibrary.wiley.com/doi/full-xml/10.1002/1873-3468.13675> DOI: 10.1002/1873-3468.13675
- POLLACK SASKIA J ET AL: "Paired Helical Filament-Forming Region of Tau (297-391) Influences Endogenous Tau Protein and Accumulates in Acidic Compartments in Human Neuronal Cells", JOURNAL OF MOLECULAR BIOLOGY, ACADEMIC PRESS, UNITED KINGDOM, vol. 432, no. 17, 16 July 2020 (2020-07-16), pages 4891 - 4907, XP086238050, ISSN: 0022-2836, [retrieved on 20200716], DOI: 10.1016/J.JMB.2020.05.027

## Description

The present invention provides a system for the study of tau protein aggregation in neuronal cells *in vitro* which can be used to screen agents for therapeutic effectiveness against aggregates of tau protein or fragments thereof.

Assembly of tau protein into paired helical filaments (PHFs) and straight filaments as aggregates is **a key feature of Alzheimer's disease (AD). Aggregation of tau has** been implicated in neurodegeneration, cellular toxicity and the propagation which accompanies disease progression. The misfolding, self-assembly and accumulation of tau protein in tangles is a major pathological feature shared by tauopathies, the most common **of which is Alzheimer's disease (AD). Under** pathological conditions, the abnormal aggregation of tau protein into paired helical filaments (PHFs) into neurofibrillary tangles (NFTs) is accompanied by the loss of function of tau protein and neuronal dysfunction.

Tau has been shown to exhibit prion-like behaviour, including cellular uptake and templated seeding. *In vitro* studies have shown that extracellular tau aggregates can be internalised by neurons to induce self-assembly of intracellular endogenous tau, which can then be released and transferred to neighbouring or synaptically connected neurons. In cultured cell lines, internalised aggregates of recombinant tau induced using heparin or AD brain-derived tau aggregates can be observed in endosomal compartments and are capable of recruiting endogenous and aggregation-prone tau to aggregate. A consistent finding in these studies is the co-localisation of tau with endosomes and lysosomes.

Whilst there is strong support for transmission of tau aggregates between neurons and/or glial cells, the species responsible for this has been debated. Studies have suggested that the seeding competence of tau is dependent on the size and conformation of the tau aggregate and that tau oligomers act as the key species for inducing propagation rather than monomers or longer fibrils purified from rTg4510 mice. It was reported in one study that large tau aggregates (>1 0mers) are the seed-competent species in P301 S tau transgenic mice, whereas in another study, tau trimers were found to be the minimal unit necessary for conformational template seeding and intracellular tau aggregation in human tau-expressing HEK-293 cells.

Many studies have used animal or cell models in which human and/or mutant tau has been overexpressed. In vitro studies have utilised full-length or truncated tau proteins that require heparin-induced fibrilisation. Both approaches aim to overcome the low aggregation propensity and the lack of cytotoxicity of full-length tau. However, there are increasing doubts as to the physiological relevance of heparin-induced tau filaments as they do not self-assemble and do not reproduce the key structural features of AD filaments. The truncated repeat-domain fragment of tau spanning residues 297-391 (referred to as dGAE (see Despres et al ACS Chem. Biol., vol. 14, no. 6, pp. 1363-1379, (2019); Zhang et al Elife, vol. 8, p. e43584, (2019); Wischik et al. Proc. Natl. Acad. Sci. USA., vol. 85, no. 12, pp. 4506-4510, (1988)) which was first identified biochemically in proteolytically stable PHF core preparations from AD brain tissues, overlaps with the core sequence (306-378) characterised by cryo-electron microscopy as forming C-shaped subunits assembled to form a combined cross-beta/beta-helix structure. It is not understood what initiates the process of tau aggregation in AD, but truncated dGAE serves for template-directed aggregation of tau in cell-free and cellular models (Harrington et al. J. Biol. Chem., vol. 290, no. 17, pp. 10862-10875, 2015), Wischik et al Proc Natl Acad Sci USA, vol. 93, no. 20, pp. 11213-11218 (1996)), and in transgenic mice (Melis et al. Cell. Mol. Life Sci., vol. 72, no. 11, pp. 2199-2222, (2015)). The dGAE region of tau (297-391) assembles spontaneously in physiological conditions to form PHF-like filaments *in vitro* in the absence of additives such as heparin.

Previous studies investigating the properties of exogenously applied tau in tissue culture cell models have been hampered by the use of non-neuronal or neuronal *in vitro* models that overexpress human wild-type or mutant tau, often using non-physiological fragments or preparations of tau to initiate aggregation (Falcon et al., J. Biol. Chem., vol. 290, no. 2, pp. 1049-1065, (2015); J. L. Guo and V. M. Y. Lee, J. Biol. Chem., vol. 286, no. 17, pp 15317-15331 (2011); and Kfoury et al., J. Biol. Chem., vol. 287, no. 23, pp. 19440-19451, (2012)).

It has also been reported that full-length tau, mutated tau and tau aggregates with different phosphorylation states have minimal effects on cell viability (Kumar et al., J. Biol. Chem., vol. 289, no. 29, pp. 20318-20332, (2014); Tepper et al., J. Biol. Chem., vol. 289, no. 49, pp. 34389-34407, (2014) and Kaniyappan et al., Alzheimer's Dement., vol. 13, no. 11, pp. 1270-1291, (2017)). For instance, it was observed that oligomers formed from a variant repeat tau fragment (corresponding to tau244-372 with a Lys-280 deletion; Tau^{RDΔK}) are selectively toxic to dendritic spines without affecting cell viability (Kaniyappan et al.,Alzheimer's Dement., vol. 13, no. 11, pp. 1270-1291, (2017)).

Other studies have also shown that toxicity is dependent the precise fragments of tau that are used (Flach et al., J. Biol. Chem., vol. 287, no. 52, pp. 43223-43233, (2012); Lasagna-Reeves et al., Biochemistry, vol. 49, no. 47, pp. 10039-10041, (2010); and Lasagna-Reeves et al., Mol. Neurodegener., vol. 6, no. 1, p. 39, (2011)). Variation in tau toxicity assays may be related in part to the differences in tau fragments used and in their methods of preparation.

Identification of Tau aggregation inhibitors is described in WO 1996/030766 and WO 2002/055720. Screening for inhibitors of paired helical filaments of Tau is described in WO 2001/018546. High-throughput screens for use in identificaton of inhobitors of Tau aggregation have been reviewed in Bulic et al (J. Med. Chem. Vol. 56, pp 4135-4155 (2013)). Liu et al (Pharm. Pat. Anal. Vol.3 pp 429-447 (2014)) also report on various Tau targetting approaches using several different mechanisms distinct from Tau aggregation inhibitors. Clinical development of compounds identified are discussed in Panza et al (BioMed Research Int., Vol. 216, Article ID 3245935, (2016)). Despite these reports none of the inhibitors other than Methylene Blue (MB) or LMT ((leuco-methylthioninium bis-hydromethanesulfonate, LMTM; also known as LMT-X or TRx0237) described have progressed to full phase III clinical trials (Panza et al (BioMed Research Int., Vol. 216, Article ID 3245935, (2016)).

The novel system described herein permits the molecular mechanisms of propagation of tau aggregation pathology to be studied in vitro in a more physiological manner with a view to development of novel therapeutic approaches. The new model system described herein permits the investigation of the internalisation, cytotoxicity and effect on endogenous tau of the PHF forming region of tau in human neuronal cells in the absence of mutant tau overexpression or exogenous seeding factors.

According to a first aspect of the present invention, there is provided a method of screening for an agent effective in inhibiting cytotoxicity of a fragment of Tau protein comprising at least 70 amino acids in the amino acid sequence of dGAE95 (SEQ ID NO:4) or a sequence with at least 85% identity thereto to a neuronal cell comprising the steps of:
(a) culturing the neuronal cell in the presence of the agent and subsequently culturing the neuronal cell with the fragment of Tau protein in the absence of heparin; or
(b) culturing the neuronal cell with the fragment of Tau protein in the absence of heparin and subsequently culturing the neuronal cell in the presence of the agent; and
(c) determining the cytotoxicity of the fragment of Tau protein to the neuronal cell after performing step (a) or step (b).

The method of the invention is suitably carried out *in vitro* on a population of neuronal cells. The methods of the invention are therefore *ex vivo* methods for screening for agents with a desired activity against aggregates of tau protein or a fragment thereof.

The neuronal cell may be cultured in the presence of the agent and then further cultured in the presence of the fragment of Tau protein. Alternatively, the neuronal cell can be cultured in the present of the fragment of Tau protein and then further cultured in the presence of the agent.

The fragments of Tau protein as defined above suitably aggregate to form self-assembled structures, including oligomers and/or filaments. The fragments of Tau protein may be in a predominantly random coil conformation and/or consist of soluble monomer and dimer forms. The aggregates of tau protein or a fragment thereof as described herein may be any suitable form of tau protein. Suitably, the tau protein is a recombinant form of tau protein. Such fragments of Tau protein may be also referred to as tau aggregates. The fragments of Tau protein may be present in a purified preparation in which contaminants (shorter peptide fragments) are removed from the preparation.

The fragments of Tau protein as described herein are able to self-aggregate without phosphorylation. The fragments of Tau protein described herein co-aggregate with endogenous Tau protein. After co-aggregation, the fragments of Tau protein described herein and the endogenous Tau protein accumulate together within endosomal and/or lysosomal compartments within a neuronal cell.

The fragments of Tau protein comprising at least 70 amino acids in the amino acid sequence of dGAE95 (SEQ ID NO:4) or a sequence with at least 85% identity thereto suitably comprises a contiguous sequence of at least 70 amino acids from the amino acid of SEQ ID NO: 4.

The fragments of Tau protein as defined above for use according to the invention may be from 70 to 97 amino acids in length, optionally from 71 to 97 amino acids in length. The fragment of Tau protein as defined above may be suitably selected from the group of fragments of 71, 73, 94, 95, 97 amino acids in length.

The fragment of Tau protein comprising at least 70 amino acids in the amino acid sequence of dGAE95 (SEQ ID NO:4) or a sequence with at least 85% identity thereto may therefore be selected from the group consisting of fragments of Tau protein having amino acid sequences as described in SEQ ID NO:4, SEQ ID NO: 3, SEQ ID NO: 5, SEQ ID NO: 6, or SEQ ID NO: 7, or a sequence with at least 85% identity thereto.

dGAE95 refers to the 95 residues fragment of Tau (2N4R) with N-terminus at residue Ile-297 and C-terminus at residue Glu-391, as described in SEQ ID NO: 4, or at homologous positions in other species (the residues mentioned referring to the human or mouse Tau sequence, which are identical in this region).

The fragments of Tau protein used according to the invention therefore also include the fragments dGAE97 (dGAE97 refers to the 97 residues fragment of Tau (2N4R) with N-terminus at residue Asp-295 and C-terminus at residue Glu-391, as described in SEQ ID NO: 3, or at homologous positions in other species), dGA ("dGA" refers to the 94 residues fragment of Tau (2N4R) with N-terminus at residue Ile-297 and C-terminus at residue Ala-390, as described in SEQ ID NO: 5, or at homologous positions in other species), dGAE73 (dGAE73 refers to the fragment of Tau (2N4R) with N-terminus at residue Val-306 and C-terminus at residue Phe-378, as described in SEQ ID NO: 6, or at homologous positions in other species), and the amino acid sequence of residues 308 to 378 of Tau (2N4R) with N-terminus at residue Ile-308 and C-terminus at residue Phe-378, as described in SEQ ID NO: 7, or at homologous positions in other species.

All residue numbers of the Tau protein sequence and structure in the present disclosure refer to the residues of SEQ ID NO:1, which is the sequence of the four-repeat isoform 2N4R of human Tau protein (Uniprot ID P10636-8), or homologous positions in other species or variants thereof. Human Tau isoform 2N4R (Uniprot ID P10636-8) corresponds to amino acids 1-124, 376-394 and 461-758 of full-length Tau, Uniprot ID P10636 or P10636-1, provided as SEQ ID NO:2. SEQ ID NO: 2 relates to a longer form of Tau found in the peripheral nervous system (PNS) but not the central nervous **system (CNS). As used herein, references to "full-length" tau refer to** SEQ ID NO: 1 (the relevant sequence for the CNS) and not to SEQ ID NO: 2 (which is not relevant in the CNS).
SEQ ID NO: 1 (Isoform Tau-F, also known as Tau-4, 2N4R, 441 amino acids): 
>sp|P10636-8|TAU_HUMAN Isoform Tau-F of Microtubule-associated protein tau OS=Homo
sapiens OX=9606 GN=MAPT








SEQ ID NO: 2 (Full length human Tau, Isoform PNS-Tau, 758 amino acids);

>sp|P10636-1|TAU_HUMAN Microtubule-associated protein tau OS=Homo sapiens OX=9606
GN=MAPT PE=1 SV=5
MAEPRQEFEVMEDHAGTYGLGDRKDQGGYTMHQDQEGDTDAGLKESPLQTPTEDGSEEPG
SETSDAKSTPTAEDVTAPLVDEGAPGKQAAAQPHTEIPEGTTAEEAGIGDTPSLEDEAAG
HVTQEPESGKVVQEGFLREPGPPGLSHQLMSGMPGAPLLPEGPREATRQPSGTGPEDTEG
GRHAPELLKHQLLGDLHQEGPPLKGAGGKERPGSKEEVDEDRDVDESSPQDSPPSKASPA
QDGRPPQTAAREATSIPGFPAEGAIPLPVDFLSKVSTEIPASEPDGPSVGRAKGQDAPLE
FTFHVEITPNVQKEQAHSEEHLGRAAFPGAPGEGPEARGPSLGEDTKEADLPEPSEKQPA
AAPRGKPVSRVPQLKARMVSKSKDGTGSDDKKAKTSTRSSAKTLKNRPCLSPKHPTPGSS
DPLIQPSSPAVCPEPPSSPKYVSSVTSRTGSSGAKEMKLKGADGKTKIATPRGAAPPGQK
GQANATRIPAKTPPAPKTPPSSGEPPKSGDRSGYSSPGSPGTPGSRSRTPSLPTPPTREP
KKVAVVRTPPKSPSSAKSRLQTAPVPMPDLKNVKSKIGSTENLKHQPGGGKVQIINKKLD
LSNVQSKCGSKDNIKHVPGGGSVQIVYKPVDLSKVTSKCGSLGNIHHKPGGGQVEVKSEK
LDFKDRVQSKIGSLDNITHVPGGGNKKIETHKLTFRENAKAKTDHGAEIVYKSPVVSGDT
SPRHLSNVSSTGSIDMVDSPQLATLADEVSASLAKQGL

dGAE97 refers to the 97 residues fragment of Tau (2N4R) with N-terminus at residue Asp-295 and C-terminus at residue Glu-391, as described in SEQ ID NO: 3, or at homologous positions in other species (the residues mentioned referring to the human or mouse Tau sequence, which are identical in this region). As will be apparent to the skilled person, dGAE97 also corresponds to the fragment of Isoform PNS-Tau (P10636-1) with N-ter at Asp-612 and C-ter at Glu-708.

SEQ ID NO: 3 (dGAE97, human/mouse, 97 amino acids):

DNIKHVPGGGSVQIVYKPVDLSKVTSKCGSLGNIHHKPGGGQVEVKSEKLDFKDRVQSKIGSLDNI
THVPGGGNKKIETHKLTFRENAKAKTDHGAE

dGAE95 refers to the 95 residues fragment of Tau (2N4R) with N-terminus at residue Ile-297 and C-terminus at residue Glu-391, as described in SEQ ID NO: 4, or at homologous positions in other species (the residues mentioned referring to the human or mouse Tau sequence, which are identical in this region). As will be apparent to the skilled person, dGAE95 also corresponds to the fragment of Isoform PNS-Tau (P10636-1) with N-ter at Ile-614 and C-ter at Glu-708. This sequence may **sometimes be referred to simply as "dGAE".** Residues 297 to 391 of Tau (2N4R) are also known as the predominant fragment isolated from proteolytically stable core of the paired helical filament (PHF).

SEQ ID NO: 4 (dGAE95 or "dGAE", human/mouse, 95 amino acids):
IKHVPGGGSVQIVYKPVDLSKVTSKCGSLGNIHHKPGGGQVEVKSEKLDFKDRVQSKIGSLDNITH
VPGGGNKKIETHKLTFRENAKAKTDHGAE

The dGAE region of tau (297-391) assembles spontaneously in physiological conditions to form PHF-like filaments *in vitro* in the absence of additives such as heparin.

**"dGA" refers** to the 94 residues fragment of Tau (2N4R) with N-terminus at residue Ile-297 and C-terminus at residue Ala-390, as described in SEQ ID NO: 5, or at homologous positions in other species (the residues mentioned referring to the human or mouse Tau sequence, which are identical in this region).

SEQ ID NO: 5 (dGA, human/mouse, 94 amino acids):
IKHVPGGGSVQIVYKPVDLSKVTSKCGSLGNIHHKPGGGQVEVKSEKLDFKDRVQSKIGSLDNITH
VPGGGNKKIETHKLTFRENAKAKTDHGA

dGAE73 refers to the fragment of Tau (2N4R) with N-terminus at residue Val-306 and C-terminus at residue Phe-378, as described in SEQ ID NO: 6, or at homologous positions in other species (the residues mentioned referring to the human or mouse Tau sequence, which are identical in this region). This fragment corresponds to residues 306-378 of the sequence identified by cryo-EM as being the core of PHFs isolated from AD brain tissue (Fitzpatrick et al, 2017; Nature). The core can extend beyond these residues but is limited by the resolution of the cryo-EM. As will be apparent to the skilled person, dGAE73 also corresponds to the fragment of Isoform PNS-Tau (P10636-1) with N-ter at Val-623 and C-ter at Phe-695.

SEQ ID NO: 6 (dGAE73, human/mouse, 73 amino acids):
VQIVYKPVDLSKVTSKCGSLGNIHHKPGGGQVEVKSEKLDFKDRVQSKIGSLDNITHVPGGGNKKI
ETHKLTF

A further fragment of the PHF core is residues 308 to 378 of Tau (2N4R) with N-terminus at residue Ile-308 and C-terminus at residue Phe-378, as described in SEQ ID NO: 7, or at homologous positions in other species (the residues mentioned referring to the human or mouse Tau sequence, which are identical in this region).

SEQ ID NO: 7 (residues 308 to 378 of 2N4R, human/mouse, 71 amino acids):
IVYKPVDLSKVTSKCGSLGNIHHKPGGGQVEVKSEKLDFKDRVQSKIGSLDNITHVPGGGNKKIET
HKLTF

One preferred form of the tau protein is the dGAE fragment of tau protein, corresponding to amino acid residues 297-391 (using numbering from 2N4R tau), suitably the recombinant form thereof, according to SEQ ID NO: 4. Following purification, dGAE exists in a predominantly random coil conformation and consists mainly of soluble monomer and dimer forms of dGAE.

The fragments of Tau which may be used in accordance with a method of the present invention are fragments comprising at least 70 amino acids in the amino acid sequence of dGAE95 (SEQ ID NO:4) or a sequence with at least 85% identity thereto. Optionally, the sequence may have 85%, 90%, 95%, 96%, 97%, 98% or 99% identity thereto.

Such sequences are therefore defined as having a certain percentage sequence identity to one or more sequences (with reference to SEQ ID NOs) as described herein. Sequence identity may be assessed by any convenient method. However, for determining the degree of sequence identity between sequences, computer programmes that make pairwise or multiple alignments of sequences are useful, for instance EMBOSS Needle or EMBOSS stretcher (both Rice, P. et al., Trends Genet., 16, (6) pp276-277, 2000) may be used for pairwise sequence alignments while Clustal Omega (Sievers F et al., Mol. Syst. Biol. 7:539, 2011) or MUSCLE (Edgar, R.C., Nucleic Acids Res. 32(5):1792-1797, 2004) may be used for multiple sequence alignments, though any other appropriate programme may be used. Whether the alignment is pairwise or multiple, it must be performed globally (i.e. across the entirety of the reference sequence) rather than locally.

Sequence alignments and percentage (%) identity calculations may be determined using for instance standard Clustal Omega parameters: matrix Gonnet, gap opening penalty 6, gap extension penalty 1. Alternatively, the standard EMBOSS Needle parameters may be used: matrix BLOSUM62, gap opening penalty 10, gap extension penalty 0.5. Any other suitable parameters may alternatively be used.

For the purposes of this application, where there is dispute between sequence identity values obtained by different methods, the value obtained by global pairwise alignment using EMBOSS Needle with default parameters shall be considered valid.

For use in the method of the invention, the aggregates of tau protein or a fragment of Tau protein (aggregates of tau protein) may suitably be diluted in a physiological buffer solution, e.g. a phosphate buffer (physiological pH, suitably pH 7.4, for example a phosphate buffered solution) and incubated at physiological temperature (suitably at 37°C) suitably with agitation prior to administration to the neuronal cell. Suitable forms of phosphate buffered solution can comprise aqueous solutions of disodium hydrogen phosphate and sodium chloride, or potassium chloride and potassium dihydrogen phosphate. The agitation may be from 500 rpm to 1000 rpm, for example 700rpm. The agitation may be for 24 hours to 96 hours, for example 24, hours, 36, hours, 48 hours, 60 hours, 72 hours, or 96 hours.

In one example, therefore a suitable preparation of the aggregates of tau protein or a fragment thereof for use in a method of the invention may be prepared by diluting 100 µM protein in 10 mM phosphate buffer (pH 7.4) and incubated at 37°C suitably with agitation, for example agitating with a speed of 700 rpm for up to 72 hours prior to administration to the neuronal cell.

The neuronal cell may suitably express the endogenous form of Tau protein. For example, the cell line SH-SY5Y expresses endogenous human Tau protein. The neuronal cell is therefore not transfected to express a Tau protein.

The neuronal cell may be from a neuronal cell line, for example a neuroblastoma cell line, e.g. the cell line SH-SY5Y. The cells may be undifferentiated and then subsequently differentiated in culture in the presence of an appropriate growth factor, for example retinoic acid. The methods of the invention may therefore use an undifferentiated neuronal cell, for example undifferentiated SH-SY5Y. Suitably, the neuronal cell is a differentiated neuronal cell, for example differentiated SH-SY5Y (dSH-SY5Y). Where the cells are an undifferentiated cell type, the methods of the invention may therefore include an initial pre-step in which the undifferentiated cells are differentiated in culture in the presence of a growth factor. The growth factor may be retinoic acid.

Suitably, the cells of the neuronal cell line may be provided in a culture medium, for example a low-serum or serum-free culture medium e.g. DMEM growth medium; or phosphate buffered saline solution, optionally supplemented with differentiation factors and/or growth factors. The neuronal cells may be cultured in such media according to the present invention.

Undifferentiated SH-SY5Y human neuroblastoma cells may be cultured in any generally suitable growth medium, for example Dulbecco's Modified Eagle Medium/Nutrient Mixture F-12 (DMEM/F-12) supplemented with 10% Foetal Calf Serum (FCS), 1% Penicillin/Streptomycin (P/S) and 1% L-glutamine. For differentiation, SH-SY5Y cells can be plated at a density of for example 25,000 to 30,000 cm², optionally 50,000 cells per well in 24-well plates or 300,000 cells per well in 6-well plates. On the first day of differentiation, media may be suitably replaced with low-serum culture media (DMEM/F-12 containing 1% Foetal Calf Serum (FCS), 1% Penicillin/Streptomycin (P/S) and 1% L-glutamine) containing 10 µM all-trans retinoic acid (RA) (Sigma-Aldrich) and cells may be incubated for 48 hours. On day 3, this process can be repeated with fresh RA and cells incubated for a further 48 hours. On day 5, the cells can be washed once in serum-free culture media to remove traces of serum. Serum-free culture media (DMEM/F-12 containing 1% P/S and 1% L-glutamine) containing 50 ng/ml brain-derived neurotrophic factor (STEMCELL Technologies) may be added to the cells and incubated for 48 hours. Differentiated cells (dSH-SY5Y) are suitably ready to use for experiments on day 7.

Incubation of the neuronal cell with the agent may be for up to 12 hours to 72 hours, for example for 12 hours, 16 hours, 20 hours, 24 hours, 36 hours, 48 hours, 60 hours, or 72 hours.

The aggregates of tau protein or a fragment thereof may be administered to the cells (plated at a density of 50,000-300,000 per well in 24-well or 6 well plates respectively) or 25,000-30,000/cm² at a concentration of from 0.01µM to 100µM, for example at 1µM, 5µM, or 10µM.

Suitably the culturing of the neuronal cell with the agent is conducted for sufficient time in order to allow the agent to be internalised by the neuronal cell, such as for example from 1-24 hours, 1 to 36 hours or 1 to 48 hours.

The administration of the aggregates of tau protein or a fragment thereof to the neuronal step is carried out under conditions to allow for the aggregates of tau protein or a fragment thereof to be internalised by the neuronal cell, such as for example from 24 to 48 hours.

Cytotoxicity with respect to the neuronal cells used in accordance with the present invention can be defined as percentage survival of cells in a culture, including the measurement of the relative oxidative stress, endogenous recruitment of tau, lysosome/mitochondrial damage in the neuronal cells, etc.

A reduction in toxicity may be determined by cell viability, cell morphology, or the cytoskeletal distribution of Tau protein in a neuronal cell.

As described herein, the agent being screened can be added to the neuronal cell at a different time point relative to the administration of the fragment of Tau protein to the neuronal cell, i.e. before administration of the fragment of Tau protein, or after the administration of the fragment of Tau protein.

The methods of the invention can therefore be used to assess whether an agent being tested is able to prevent or reduce the cytotoxicity of the fragment of Tau protein as defined above. A reduction in the cytotoxicity of the fragment of Tau protein may be shown in the reduction in the percentage of cell death in a culture of neuronal cells cultured with a fragment of Tau protein as defined above in the presence of an agent compared to the percentage of cell death in a culture of neuronal cells cultured with a fragment of Tau protein as defined above in the absence of the agent.

Suitably, the determination of the inhibition of cytotoxicity in step (c) can include a comparison with a control value for the neuronal cell when the neuronal cell is incubated with aggregates of tau protein or a fragment thereof in the absence of the agent being tested. The determination of cytotoxicity can be expressed as a percentage of control-treated cells (neuronal cells incubated with a fragment of Tau protein as defined above in the absence of the agent). A reference level for the toxicity of the fragment of Tau protein as defined above can also be established with reference to buffer-treated cells which will provide the level of absolute toxicity for the concentration of the fragment of Tau protein as defined above used in any given assay.

A suitable method to establish whether the result of an assay showed that an agent was able to prevent or reduce cytotoxicity would be by means of a statistical test, i.e. a statistical test to examine significance of differences (e.g. unpaired T test). The reduction may therefore be a statistically significant reduction in cytotoxicity. For example, a cytotoxic fragment of Tau protein may result in 40%-60% cell death in a culture of neuronal cells compared to a control value of 20% cell death for a neuronal cell culture in a control culture medium, for example a low-serum or serum-free culture medium e.g. DMEM growth medium; or phosphate buffered saline solution.

The assay for cytotoxicity according to a method of the invention may be suitably normalised to the results obtained for cells treated without agent or with treatment vehicle. Compounds exhibiting 50% inhibition with agent at a suitable concentration of about 2 µM or less may be considered as active inhibitors of cytotoxicity. The relative activity can also therefore be compared for different agents also according to a method of the invention as described herein.

The cytotoxicity of any substance can be assayed by means of a cell viability assay. The cell viability assay may be undertaken using any generally suitable method, such as for example (i) a fluorometric assay; (ii) a colorimetric assay; (iii) a luminometric assay; or (iv) a dye exclusion assay.

Fluorometric assays for cytotoxicity include: ReadyProbes^{®} Cell Viability Imaging Kit (Life Technologies), resazurin, alamarBlue assay or CFDA-AM assay. Colorimetric assays for cytotoxicity include: MTT assay, MTS assay, XTT assay, WST-1 assay, WST-8 assay, LDH assay, SRB assay, NRU assay or crystal violet assay. Luminometric assays for cytotoxicity include: ATP assay or real-time viability assay. Dye exclusion assays for cytotoxicity include: TUNEL, Caspase-3, Trypan blue, eosin, Congo red, erythrosine B assays.

Fluorometric assays of cell viability and cytotoxicity are easy to perform with the use of a fluorescence microscope, fluorometer, fluorescence microplate reader or flow cytometer, and they offer many advantages over traditional dye exclusion and colorimetric assays. Fluorometric assays are also applicable for adherent or suspended cell lines and easy to use. A fluorometric assay may therefore be preferred in assessing the effect of an agent of interest on the cytotoxicity of aggregates of tau protein or a fragment thereof on neuronal cells according to a method of the invention.

One such approach using a fluorometric approach may suitably use a differential fluorescence approach. Following the addition of aggregates of tau protein or a fragment thereof to a population of neuronal cells, e.g. dGAE, cell viability may be measured using ReadyProbes^{®} Cell Viability Imaging Kit (Life Technologies). The kit contains a NucBlue^{®} reagent which can be used to label all cells (blue) and a NucGreen^{®} reagent to label dead cells only (green). For tagged aggregates of tau protein or a fragment thereof, e.g. dGAE, NucRed^{®} reagent can be used to label dead cells (red).

Neuronal cells may suitably be incubated with the reagents at 37°C for 15 minutes. DAPI fluorescence may be captured using a G 365 excitation filter and a LP 420 emission filter with a FT 395 dichroic. Green fluorescence may be captured using a FITC filter set (BP 450-490 excitation filter, BP 515-565 emission filter and FT 510 dichroic).

The proportion of buffer-treated cell death may be quantified by converting the images to grayscale followed by manually adjusting the threshold and converting it into a binary image to highlight live DAPI-stained cells. The number of cells may be automatically counted. The dead cells may be counted in the same way and cell death can expressed as a percentage of buffer-treated cells.

Fluorescent activated cell sorting (FACS) may also be used for bulk measurements of fluorescence intensity. A quantification of internalised unlabelled fragments of Tau protein can be determined using immunofluorescence. Alternatively, unlabelled fragments of Tau protein can be measured using thioflavin S (ThS) staining or similar.

A colorimetric assay measures a biochemical marker to evaluate metabolic activity of a cell. Reagents used in colorimetric assays develop a colour in response to the viability of cells, allowing the colorimetric measurement of cell viability via spectrophotometer. Colorimetric assays are applicable for adherent or suspended cell lines.

Luminometric assays provide fast and simple determination of cell proliferation and cytotoxicity in mammalian cells. These assays can be performed in a convenient 96-well and 384-well microtiter plate format and detection by luminometric microplate reader.

The simplest and widely method for assessing cytotoxicity is the dye exclusion method. In a dye exclusion method, viable cells exclude dyes, but dead cells not exclude them. A variety of such dyes may be used, including eosin, Congo red, erythrosine B, and trypan blue.

The system of the invention can be used to assess the effectiveness of an agent of interest in altering the effect of aggregates of tau protein or a fragment thereof on a neuronal cell. The agent may be any pharmaceutically active molecule or substance, including compounds or biological molecules, suitably a specific binding molecule, such as antibodies (for example, polyclonal antibodies or monoclonal antibodies, including bispecific antibodies, or antibody fragments such as Fab, F(ab')₂, Fc, Fd, Fv, dAb, scFv, CDR fragment, diabody, or variants or derivatives thereof). The agent may be administered directly or formulated in a suitable adjuvant, diluent or solution (e.g. a suitable physiologically buffered solution, such as phosphate buffered saline), or formulated as a pharmaceutical formulation using standard techniques know in the art.

The methods of the invention may comprise a step of establishing the activity of an agent as a positive control with known activity. Examples of known test compounds with established inhibitory activity against fragments of Tau protein for use as a positive control may include, but are not limited to, Methylene Blue (MB; also known as methylthioninium chloride) or LMT (leuco-methylthioninium bis-hydromethanesulfonate, LMTM; also known as LMT-X or TRx0237). The activity of an agent assayed according to a method of the invention can be compared to the positive control.

The methods of the invention may comprise a step of establishing a baseline for no activity of an agent using a negative control (with no known activity). Examples of suitable negative controls may include culture medium for the neuronal cells, phosphate buffered saline solution, or similar. The activity of an agent assayed according to a method of the invention can be compared to the negative control.

Optionally, the method of the invention may additionally comprise the step of treating a subject with a tauopathy with the said agent, further optionally including another therapeutically active substance suitable for the treatment of a tauopathy.

The present invention therefore provides a system for study of the effects of agents on the behaviour of aggregates of tau protein or a fragment thereof which can be used to assess the effectiveness of the agent in the treatment of a tauopathy or disease characterised by abnormal accumulation of tau proteins in neuronal cells of a subject.

Aggregation of the tau protein is a hallmark of diseases referred to as "tauopathies". Various tauopathy disorders that have been recognized which feature prominent tau pathology in neurons and/or glia and this term has been used in the art for several years. The similarities between these pathological inclusions and the characteristic tau inclusions in diseases such as AD indicate that the structural features are shared and that it is the topographic distribution of the pathology that is responsible for the different clinical phenotypes observed. In particular, cryo-electron microscope structures of aggregated Tau in AD, Pick's disease (a subtype of Frontotemporal Dementia), chronic traumatic encephalopathy (CTE) and cortico-basal degeneration (CBD) have been previously obtained, and all show common conformational features, indicating that compounds that have the ability to modulate Tau aggregation in e.g. PHFs (as observed in AD), may also modulate aggregation of Tau in other tauopathies. In addition to specific diseases discussed below, those skilled in the art can identify tauopathies by combinations of cognitive or behavioural symptoms, plus additionally through the use of appropriate ligands for aggregated tau as visualised using PET or MRI, such as those described in WO 02/075318.

As well as Alzheimer's disease (AD), the pathogenesis of neurodegenerative disorders such as Pick's disease and Progressive Supranuclear Palsy (PSP) appears to correlate with an accumulation of pathological truncated tau aggregates in the dentate gyrus and stellate pyramidal cells of the neocortex, respectively. Relevant tauopathies (or tau-associated dementias) include fronto-temporal dementia (FTD); parkinsonism linked to chromosome 17 (FTDP-17); disinhibition-dementia-parkinsonism-amyotrophy complex (DDPAC); pallido-ponto-nigral degeneration (PPND); Guam-ALS syndrome; pallido-nigro-luysian degeneration (PNLD); cortico-basal degeneration (CBD); Dementia with Argyrophilic grains (AgD); Dementia pugilistica (DP) wherein despite different topography, NFTs are similar to those observed in AD (Bouras et al., 1992); Chronic traumatic encephalopathy (CTE), a tauopathy including DP as well as repeated and sports-related concussion (McKee, et al., 2009). Others are discussed in Wischik et al. 2000, for detailed discussion - especially Table 5.1).

Abnormal tau in NFTs is found also in Down's Syndrome (DS) (Flament et al., 1990), and in dementia with Lewy bodies (DLB) (Harrington et al., 1994). Tau-positive NFTs are also found in Postencephalitic parkinsonism (PEP) (Charpiot et al., 1992). Glial tau tangles are observed in Subacute sclerosing panencephalitis (SSPE) (Ikeda et al., 1995). Other tauopathies include Niemann-Pick disease type C (NPC) (Love et al., 1995); Sanfilippo syndrome type B (or mucopolysaccharidosis III B, MPS III B) (Ohmi, et al., 2009); myotonic dystrophies (DM), DM1 (Sergeant, et al., 2001 and references cited therein) and DM2 (Maurage et al., 2005). Additionally, there is a growing consensus in the literature that a tau pathology may also contribute more generally to cognitive deficits and decline, including in mild cognitive impairment (MCI) (see e.g. Braak, et al., 2003, Wischik et al., 2018).

All of these diseases, which are characterized primarily or partially by abnormal tau aggregation, are referred to herein as "tauopathies" or "diseases of tau protein aggregation". In aspects of the present invention relating to tauopathies, the tauopathy may be selected from any tauopathy defined herein.

The tauopathy may be selected from the group consisting of Alzheimer's disease, Primary age-related tauopathy (PART), Neurofibrillary tangle-predominant senile dementia, Chronic traumatic encephalopathy (CTE), Progressive supranuclear palsy (PSP), Corticobasal degeneration (CBD), Frontotemporal dementia (FTD), Frontotemporal dementia and parkinsonism linked to chromosome 17 (FTDP-17), Pick disease, disinhibition-dementia-parkinsonism-amyotrophy complex (DDPAC), pallido-ponto-nigral degeneration (PPND), Guam-ALS syndrome; pallido-nigro-luysian degeneration (PNLD), Dementia with Argyrophilic grains (AgD), Down's Syndrome (DS), dementia with Lewy bodies (DLB), Postencephalitic parkinsonism (PEP), Dementia pugilistica (DP), traumatic brain injury (TBI), stroke, ischemia, Lytico-bodig disease (Parkinson-dementia complex of Guam), Ganglioglioma, Gangliocytoma, Meningioangiomatosis, Postencephalitic parkinsonism, Subacute sclerosing panencephalitis (SSPE), Lead encephalopathy, tuberous sclerosis, Pantothenate kinase-associated neurodegeneration, lipofuscinosis and mild cognitive impairment (MCI).

The tauopathy may be Alzheimer's disease.

Described herein is also treatment as a prophylactic measure. The treatment may be prophylactic treatment. The treatment may be by active immunization or passive immunization.

Active tau immunization has been shown to reduce tau pathology by targeting single or multiple phospho-epitopes, the amino terminus, full-length normal and mutant tau or aggregated tau. Reductions in pathological tau are achieved with few reported adverse effects, and the long-lasting immune response makes active immunization a promising option. However, elicitation of antibodies against a native protein always carries the risk of adverse immune reactions and detrimental targeting of the normal protein.

Passive immunization offers a potential solution to the safety concerns that arise from active strategies. Patients will not develop their own antibodies, and the effects of immunization are likely to be transient, which reduces the risk of immunological adverse effects. Passive immunization also offers greater specificity for the epitope that is being targeted. Antibodies could also modify disease progression by blocking the spread of tau pathology.

The agents identified by a method of the present invention with therapeutic effectiveness against a tauopathy or disease characterised by abnormal accumulation of tau proteins in neuronal cells of a subject may therefore be for use in treatment of an early stage tauopathy and/or a tauopathy characterised by mild symptoms. The agent may be for use in treatment of mild cognitive impairment (MCI).

The agent may be for use in treatment of a tauopathy in a subject at risk of developing a tauopathy. The subject at risk of developing a tauopathy may be identified by any suitable means, such as one or more of medical history, physical examination, neurological examination, brain imaging, mental status tests (such as the Mini-Mental State Exam (MMSE) and the Mini-Cog test), computerised cognitive tests (such as the Cantab Mobile, Cognigram, Cognivue, Cognision and Automated Neuropsychological Assessment Metrics (ANAM) devices), mood assessment and genetic testing. The skilled person is aware that a tauopathy diagnosis may not always be definitive until post-mortem. Accordingly, the agent may be for use in treatment of a tauopathy in a subject at risk of developing a tauopathy. The subject may be suspected of having a tauopathy. The subject may have one or more symptoms of a tauopathy. The agent may be for use in slowing progression of a tauopathy or suspected tauopathy.

The term "therapeutically-effective amount," where used herein, pertains to that amount of an agent used in the practice of the combination methodologies of the invention which is effective for producing some desired therapeutic effect, commensurate with a reasonable benefit/risk ratio, when administered in accordance with a desired treatment regimen.

Described herein but not claimed is a method of prophylactic treatment of a tauopathy in a subject, which method comprises administering to said subject an agent identified by a method of the invention.

The term "prophylactically effective amount," where used herein, pertains to that amount of an agent identified by a method of the invention which is effective for producing some desired prophylactic effect, commensurate with a reasonable benefit/risk ratio, when administered in accordance with a desired treatment regimen. "Prophylaxis" in the context of the present specification should not be understood to circumscribe complete success i.e. complete protection or complete prevention. Rather prophylaxis in the present context refers to a measure which is administered in advance of detection of a symptomatic condition with the aim of preserving health by helping to delay, mitigate or avoid that particular condition.

Preferred features of the second and subsequent aspects of the invention are as for the first aspect *mutatis mutandis.*

According to a second aspect of the present invention, there is provided a method of screening for an agent effective in inhibiting internalisation of a fragment of Tau protein comprising at least 70 amino acids in the amino acid sequence of dGAE95 (SEQ ID NO:4) or a sequence with at least 85% identity thereto comprising the steps of:
(a) culturing the neuronal cell in the presence of the agent and subsequently culturing the neuronal cell with the fragment of Tau protein in the absence of heparin; or;
(b) culturing the neuronal cell of with the fragment of Tau protein in the absence of heparin and subsequently culturing the neuronal cell in the presence of the agent; and
(c) determining the internalisation of the fragment of Tau protein in the neuronal cell after performing step (a) or step (b).

As described herein, internalisation of the fragment of Tau protein as defined above results in production of insoluble tau species (i.e. aggregates), abnormal phosphorylation and truncation of endogenous full-length tau protein.

Inhibition of internalisation of a fragment of Tau protein as described herein may be assayed using immunofluorescence and/or transmission electron microscopy (TEM). Such techniques can show the relative amounts of the fragment of Tau protein internalised by the neuronal cells in the presence of an active agent. Suitable positive and negative controls can be prepared also as described herein in order to establish known level of activity for an agent with known inhibitory activity and baseline activity in the absence of agent, respectively.

In a method using immunofluorescence, the fragments of Tau protein as described herein may be labelled with a detectable fluorescent label. The method can use a single (primary) antibody, linked to a suitable fluorophore wherein the antibody specifically recognizes and binds to a fragment of Tau protein as described herein. Alternatively, the method can use two antibodies, in which an unlabelled first (primary) antibody specifically recognizes and binds to a fragment of Tau protein as described herein, and a secondary antibody labelled with a fluorophore recognise and binds the primary antibody.

Immunogold transmission electron microscopy (TEM) uses colloidal gold particles which may be attached to antibodies which specifically recognise and bind a fragment of Tau protein as defined herein. The colloidal gold particles may be attached to a primary antibody which specifically recognised and binds to a fragment of Tau protein as defined herein, or the colloidal gold particles may be attached to secondary antibody which specifically recognises and binds to a primary antibody which specifically recognises and binds to a fragment of Tau protein as described herein.

Internalisation of a fragment of Tau protein as described herein may also be assayed using a detectably labelled fragment of Tau protein, for example a fluorescently labelled fragment of Tau protein.

The step of determining the inhibition of internalisation of the fragment of Tau protein in the neuronal cell may therefore use photography or TEM in order to visualise the relative amount of Tau protein fragment internalised in the neuronal cells.

Immunofluorescence and immunogold transmission electron microscopy (TEM) of treated cells as described herein reveal that the fragment of Tau protein as defined above, e.g. for example dGAE, accumulates within the endosomal/lysosomal compartments of cells.

Following incubation, the neuronal cells can be imaged and analysis performed using a suitable analysis program, such as for example "imaged", to quantify the fluorescence intensity within the cell body to provide a readout of internalisation and statistical analysis carried out to compare fluorescence intensity of positive and negative controls with agent treated samples. The number of cells can be counted to express the level of internalisation as mean fluorescence intensity/cell to take into account different numbers of cells in fields of view.

A suitable method to establish whether the result of an assay showed that an agent was able to inhibit internalisation of a fragment of Tau protein may be undertaken by means of a statistical test, i.e. a statistical test to examine significance of differences (e.g. unpaired T test). The reduction may therefore be a statistically significant reduction in internalisation. For example, a fragment of Tau protein able to serve as an inhibitor of internalisation may result in 40%-60% inhibition of internalisation of the fragment of Tau protein in a culture of neuronal cells compared to a control value of 20% inhibition for a neuronal cell culture in a control culture medium, e.g. DMEM growth medium.

The assay for inhibition of internalisation of a fragment of Tau protein according to a method of the invention may be suitably normalised to the results obtained for cells treated without agent or with treatment vehicle. Compounds exhibiting 50% inhibition with agent at a suitable concentration of about 2 µM or less may be considered as active inhibitors of internalisation. The relative activity can also therefore be compared for different agents also according to a method of the invention as described herein.

The methods of the invention may comprise a step of establishing the activity of an agent as a positive control with known activity. Examples of known test compounds with established inhibitory activity against fragments of Tau protein for use as a positive control may include, but are not limited to, Methylene Blue (MB also known as methylthioninium chloride) or LMT (leuco-methylthioninium bis-hydromethanesulfonate, LMTM; also known as LMT-X or TRx0237). The activity of an agent assayed according to a method of the invention can be compared to the positive control.

The methods of the invention may comprise a step of establishing a baseline for no activity of an agent using a negative control (with no known activity). Examples of suitable negative controls may include culture medium for the neuronal cells, phosphate buffered saline solution, or similar. The activity of an agent assayed according to a method of the invention can be compared to the negative control.

Optionally, the method described herein may additionally comprise the step not part of the invention of treating a subject with a tauopathy with the said agent, further optionally including another therapeutically active substance suitable for the treatment of a tauopathy.

According to a third aspect of the present invention, there is provided a method of screening for an agent effective in disrupting interaction of a fragment of Tau protein comprising at least 70 amino acids in the amino acid sequence of dGAE95 (SEQ ID NO:4) or a sequence with at least 85% identity thereto with endogenous tau protein comprising the steps of:
(a) culturing the neuronal cell in the presence of the agent and subsequently culturing the neuronal cell with the fragment of Tau protein in the absence of heparin; or;
(b) culturing the neuronal cell with the fragment of Tau protein in the absence of heparin and subsequently culturing the neuronal cell in the presence of the agent; and
(c) determining the extent of interaction of the fragment of Tau protein with endogenous tau protein in the neuronal cell after performing step (a) or step (b).

The disruption of the interaction between the fragment of Tau protein and the endogenous Tau protein may be suitably assayed using immunofluorescence and/or immunogold transmission electron microscopy (TEM) as described herein.

As described herein, internalisation of the fragment of Tau protein as defined above results in production of insoluble tau species, abnormal phosphorylation and truncation of endogenous full-length tau protein.

Immunofluorescence and immunogold TEM of treated cells reveal that the aggregates of tau protein or a fragment thereof, e.g. for example dGAE, accumulates within the endosomal/lysosomal compartments of cells. Disruption of the interaction of a fragment of Tau protein with endogenous Tau protein may be described as inhibition of the interaction between the fragment of Tau protein and the endogenous Tau protein.

The step of determining the disruption of the interaction between the fragment of Tau protein and the endogenous Tau protein in the neuronal cell may therefore use photography or TEM in order to visualise the relative interaction of Tau protein fragment and endogenous Tau protein in the neuronal cells.

As described herein, immunofluorescence and/or immunogold TEM can be used to show the relative disruption of the interaction between a fragment of Tau protein and endogenous Tau protein internalised by the neuronal cells in the presence of an active agent. Suitable positive and negative controls can be prepared also as described herein in order to establish known level of activity for an agent with known inhibitory activity and baseline activity in the absence of agent, respectively.

A suitable method to establish whether the result of an assay showed that an agent was able to disrupt interaction of a fragment of Tau protein with endogenous Tau protein may be undertaken by means of a statistical test, i.e. a statistical test to examine significance of differences (e.g. unpaired T test). The reduction may therefore be a statistically significant reduction in disruption of the interaction of a fragment of Tau with endogenous Tau protein. For example, a fragment of Tau protein able to serve as a disruption of interaction may result in 40%-60% disruption of the interaction of the fragment of Tau protein with endogenous Tau protein in a culture of neuronal cells compared to a control value of 20% inhibition for a neuronal cell culture in a control culture medium, e.g. DMEM growth medium.

The assay for disruption of interaction of a fragment of Tau protein with endogenous Tau protein according to a method of the invention may be suitably normalised to the results obtained for cells treated without agent or with treatment vehicle. Compounds exhibiting 50% activity with agent at a suitable concentration of about 2 µM or less may be considered as being active inhibitors of the interaction. The relative activity can also therefore be compared for different agents also according to a method of the invention as described herein.

The methods of the invention may comprise a step of establishing the activity of an agent as a positive control with known activity. Examples of known test compounds with established inhibitory activity against fragments of Tau protein for use as a positive control may include, but are not limited to, Methylene Blue (MB also known as methylthioninium chloride) or LMT (leuco-methylthioninium bis-hydromethanesulfonate, LMTM; also known as LMT-X or TRx0237). The activity of an agent assayed according to a method of the invention can be compared to the positive control.

The methods of the invention may comprise a step of establishing a baseline for no activity of an agent using a negative control (with no known activity). Examples of suitable negative controls may include culture medium for the neuronal cells, phosphate buffered saline solution, or similar. The activity of an agent assayed according to a method of the invention can be compared to the negative control.

Optionally, the method described herein may additionally comprise the step not part of the invention of treating a subject with a tauopathy with the said agent, further optionally including another therapeutically active substance suitable for the treatment of a tauopathy.

According to a fourth aspect of the present invention, there is provided a system for screening for an agent effective in inhibiting cytotoxicity of a fragment of Tau protein comprising at least 70 amino acids in the amino acid sequence of dGAE95 (SEQ ID NO:4) or a sequence with at least 85% identity thereto comprising (i) a neuronal cell line. (ii) a fragment of Tau protein comprising at least 70 amino acids in the amino acid sequence of dGAE95 (SEQ ID NO:4) or a sequence with at least 85% identity thereto; and (iii) a library of agents; wherein the neuronal cell line is free from heparin.

The library of agents may comprise any agent of interest as described herein which can alter the effect of aggregates of tau protein or a fragment thereof as described herein on a neuronal cell. The agent may be any pharmaceutically active molecule or substance, including compounds or biological molecules, suitably a specific binding molecule, such as antibodies. The library may be a library of chemical compounds or a library of specific binding molecules.

Such a system of the invention may be provided in the form of a kit of parts comprising a population of cells of a neuronal cell line; a fragment of Tau protein comprising at least 70 amino acids in the amino acid sequence of dGAE95 (SEQ ID NO:4) or a sequence with at least 85% identity thereto; and a library of agents; wherein the neuronal cell line is free from heparin. Such a system or kit of parts of the invention may also be provided with instructions for use. The cells may be provided in a suitable growth or culture medium as described herein. The cells may be provided in a suitable container also,

Other preferred features of the fourth and subsequent aspects of the invention relating to systems of the invention are as described above.

According to a fifth aspect of the present invention, there is provided a system for screening for an agent effective in inhibiting internalisation of a fragment of Tau protein comprising at least 70 amino acids in the amino acid sequence of dGAE95 (SEQ ID NO:4) or a sequence with at least 85% identity thereto comprising (i) a neuronal cell line. (ii) a fragment of Tau protein comprising at least 70 amino acids in the amino acid sequence of dGAE95 (SEQ ID NO:4) or a sequence with at least 85% identity thereto; and (iii) a library of agents; wherein the neuronal cell line is free from heparin.

The library of agents may comprise any agent of interest as described herein which can alter the effect of aggregates of tau protein or a fragment thereof as described herein on a neuronal cell. The agent may be any pharmaceutically active molecule or substance, including compounds or biological molecules, suitably a specific binding molecule, such as antibodies. The library may be a library of chemical compounds or a library of specific binding molecules.

Such a system of the invention may be provided in the form of a kit of parts comprising a neuronal cell line; a fragment of Tau protein comprising at least 70 amino acids in the amino acid sequence of dGAE95 (SEQ ID NO:4) or a sequence with at least 85% identity thereto; and a library of agents; wherein the neuronal cell line is free from heparin.

According to a sixth aspect of the present invention, there is provided a system for screening for an agent effective in disrupting interaction of a fragment of Tau protein comprising at least 70 amino acids in the amino acid sequence of dGAE95 (SEQ ID NO:4) or a sequence with at least 85% identity thereto with endogenous tau protein comprising (i) a neuronal cell line. (ii) a fragment of Tau protein comprising at least 70 amino acids in the amino acid sequence of dGAE95 (SEQ ID NO:4) or a sequence with at least 85% identity thereto; and (iii) a library of agents; wherein the neuronal cell line is free from heparin.

The library of agents may comprise any agent of interest as described herein which can alter the effect of aggregates of tau protein or a fragment thereof as described herein on a neuronal cell. The agent may be any pharmaceutically active molecule or substance, including compounds or biological molecules, suitably a specific binding molecule, such as antibodies. The library may be a library of chemical compounds or a library of specific binding molecules.

Such a system of the invention may be provided in the form of a kit of parts comprising a neuronal cell line; a fragment of Tau protein comprising at least 70 amino acids in the amino acid sequence of dGAE95 (SEQ ID NO:4) or a sequence with at least 85% identity thereto; and a library of agents; wherein the neuronal cell line is free from heparin.

The present disclosure describes how the cellular uptake of soluble and fragments of Tau protein as defined herein, i.e. aggregated forms of the tau protein, for example the tau protein fragment dGAE, can be explored. The downstream consequences of tau internalisation into differentiated SH-SY5Y neuroblastoma cells can be examined using fluorescence and electron microscopy alongside structural and biochemical analyses.

In an embodiment of a method or system of any aspect of the invention, undifferentiated neuronal cells may be plated in a multi-well plate at a suitable concentration (for example, 30,000 cells per well) and either differentiated using retinoic acid in depleted serum medium, as described previously, or left undifferentiated. Cells can be then treated in one of the following ways:
either (1) the cells are pre-incubated in the presence of an agent of interest prior to adding fragments of Tau protein as defined herein (such fragments may be also referred to as tau aggregates); or (2) the cells are co-incubated with an agent of interest and tau aggregates administered at the same time.

The cells may be incubated for 12, 24, or 48 hours at each stage. For example, the cells may be incubated for 24 hours meaning the total time for (1) is 48 hours and 24 hours for (2). For example, as described herein, the tau protein fragment dGAE-488 (for example used at a concentration of 1 µM) can be incubated with cells for 24 hours which is sufficient for internalised tau protein to be seen.

Following incubation, cells may be photographed and analysis of images performed using a suitable analysis software program (for example imaged) to quantify the fluorescence intensity within the cell body. The quantification of fluorescence intensity can provide a measure of tau protein internalisation.

The number of cells may be counted to express the level of internalisation as mean fluorescence intensity/cell to take into account different numbers of cells in fields of view.

In one embodiment, a fragment of Tau protein, for example dGAE-488, can be added at a suitable concentration, for example about 1 µM. Agents for screening can be tested initially at a single concentration, for example 10 µM; those agents showing inhibitory activity may then be tested over at a range of concentrations, e.g. for example of from about 2 µM down to about 20 nM and the activity of compounds reported as the concentration at which, for example, 50% inhibition of activity is observed.

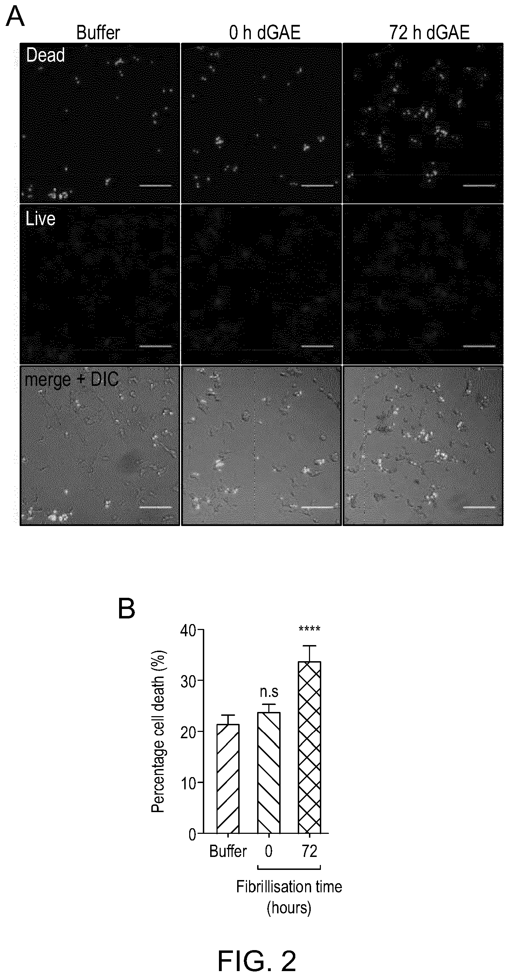

The activities measured according to a method or system of the invention may include, as described herein: (i) internalisation of tau (for example as measured by intracellular fluorescence of a detectably labelled fragment of Tau protein as described herein); (ii) intracellular aggregation of a detectably labelled fragment of Tau protein as described herein (for example as measured by harvesting cells, lysing them and separating aggregated labelled fragments of Tau protein by ultracentrifugation and measuring aggregated tau (for example by immunoblot with anti-tau antibody); and (iii) cytotoxicity of a fragment of Tau protein as described herein (for example as measured using a suitable cytotoxicity assay (for example the ReadyProbes^{®} assay) in order to measure cell death (see for example figure 2A).

The activities may be normalised to the results obtained for cells treated without agent or with treatment vehicle. Suitably, compounds exhibiting 50% inhibition with an agent tested at 2 µM or less being considered active. The methods and systems described herein also permit relative activity to be compared for different agents.

The results of the studies described herein show that assembled dGAE exhibits more acute cytotoxicity than the soluble, non-aggregated form. Conversely, the soluble form is much more readily internalised and, once within the cell, is able to associate with endogenous tau resulting in increased phosphorylation and aggregation of endogenous tau, which accumulates in lysosomal/endosomal compartments of the cell. It appears that soluble oligomeric forms are able to propagate tau pathology without being acutely toxic. The studies described herein therefore provide methods and systems for determining the activity of agents in inhibiting the activity of a fragment of Tau protein as measured by cytotoxicity of a fragment of Tau protein as described herein, internalisation of a fragment of Tau protein as described herein and/or the interaction of endogenous Tau protein with a fragment of Tau protein as described herein.

The results presented herein show that aggregated dGAE is cytotoxic while the soluble, non-fibrillar dGAE is not acutely toxic and is taken up by neuron-like cells. Internalisation results in production of insoluble tau species, abnormal phosphorylation and truncation of endogenous full-length tau. Immunofluorescence and immunogold TEM of treated cells reveal that dGAE accumulates within the endosomal/lysosomal compartments of cells. The downstream consequences of internalisation of Tau fragments are therefore cytotoxicity, aggregation into insoluble tau species and truncation/phosphorylation of tau.

In the 24-hourtimeframe of the experiments described herein, an increase in cell death was observed following exposure of differentiated neuroblastoma cells to aggregated dGAE but minimal differences in the viability of cells treated with soluble dGAE.

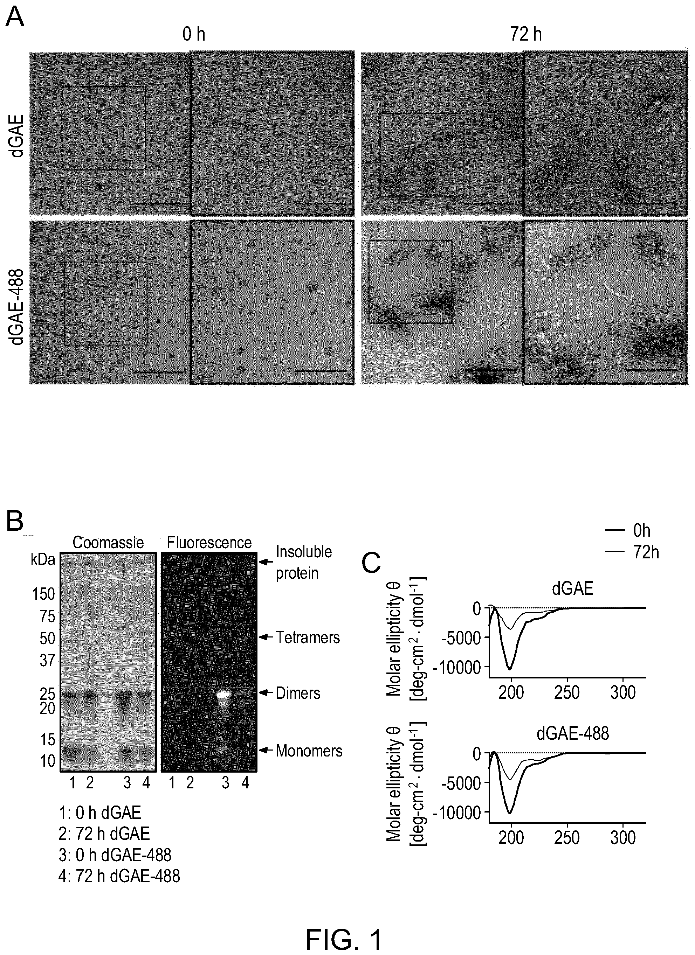

Under conditions used here, aggregated dGAE appeared to be more acutely toxic to differentiated neuroblastoma cells following the 24h incubation period. Although minimally toxic even at higher concentrations, the dGAE in its soluble form was nevertheless internalised into differentiated neuroblastoma cells within 2-h exposure. After agitation, the dGAE-488 is internalised much less efficiently. In the present study, some internalisation using aggregated preparations was observed but these may be soluble or partially soluble species that remain in solution during preparation of filaments. It is likely that the agitated dGAE-488 contains a mixture of sizes of tau assemblies, some of which are internalised and some of which are not. Whilst the exact species being internalised remains to be resolved, the soluble dGAE preparation consists of aggregates ranging in diameter from 10-80 nm implying a manifold of oligomeric forms (Fig 1A).

Furthermore, the time taken for 0h dGAE to become toxic may be longer than the timeframe of these experiments. The internalisation described herein does not require exogenous factors either for aggregation or for cellular uptake.

The present studies have investigated whether endogenous tau could be recruited and converted into insoluble forms following uptake of soluble dGAE into the cytoplasm. The interaction of between endocytosed tau with cytosolic tau has been investigated previously in various cell models that have required the overexpression of human tau, or by introducing exogenous tau with the help of protein delivery reagents.

The data presented herein shows that incubation with soluble forms of dGAE results in a local increase in endogenous phospho-tau in cells with normal levels of endogenous tau, consistent with tau pathology in AD. Using dGAE-488 together with antibodies recognising epitopes outside the repeat domain to probe the phosphorylation state of endogenous tau protein, it has been shown herein that there is an increase in phosphorylation of endogenous tau at T231 and S202-T205. These changes are accompanied by an increase in levels of insoluble phosphorylated tau protein after sequential extraction, but not in the whole lysate. Western blots from whole lysate did not reveal any clear differences in phospho-tau levels using AT80 or AT180 which may suggest that overall levels remain constant, but that local phospho-tau levels increase within areas of insoluble/aggregated tau as shown by immunofluorescence and following sequential extraction. The mechanism of induction of aggregation or increased phosphorylation following uptake of soluble forms of dGAE is not known at this stage. This truncated tau species is able to self-aggregate spontaneously without any requirement for phosphorylation. Here we show that dGAE co-aggregates with endogenous tau and that these accumulate together within endosomal/lysosomal compartments.

The present disclosure shows that the majority of dGAE that is internalised by cells is localised to lysosomal compartments. Using immunogold TEM, the localisation of dGAE-488 to vesicular structures has been demonstrated with a size range suggestive of lysosomal compartments.

The immunofluorescence and TEM findings together suggest the accumulation of both dGAE and endogenous tau within the endo-lysosomal compartments. These findings provide a novel perspective on the ultrastructure of cells following internalisation of a pathological form of tau and suggests that the autophagy pathway may be implicated in its degradation.

Physiological and pathological forms of tau are cleared by the proteasomal and autophagic degradative systems and it has been suggested that pathological tau is preferentially degraded by the autophagic-lysosomal system. It is possible that the accumulation of pathological tau may interfere with the normal functioning of these degradative processes. In AD, there is support for impaired degradation of autophagic vacuoles by lysosomes suggesting that incomplete clearance of pathological tau could contribute to neuronal dysfunction. Understanding how accumulated tau species can be cleared from cells may allow for the development of complementary therapeutic approaches in addition to inhibition of tau aggregation.

The ease with which soluble dGAE self-assembles and internalises into neuronal cells makes it possible to study the effect of its self-assembly in a cellular environment, in particular its effect on acute toxicity and its influence on phosphorylation state and insolubility of endogenous tau at an ultrastructural level. This approach will provide a useful tool to facilitate the study of tau aggregation in the absence of any overexpression of the protein, post-translational modification, or inducers of assembly and ultimately to study the mechanism of action of tau aggregation inhibitors that target tau seeding and neuronal transmission in AD and related tauopathies.

As stated above, preferred features of the second and subsequent aspects of the invention are as for the first aspect *mutatis mutandis.*

The present invention will now be further described with reference to the following Examples which are included for the purposes of reference only and should not be construed as being limitations on the invention, reference is also made to a number of figures in which:
FIGURE 1 shows dGAE and dGAE-488 self-assemble to form structurally similar fibrils. dGAE and dGAE-488 were incubated at 100 µM for 72 h. Aliquots of assembly mixture were taken before (0 h) and after (72 h) fibrillisation for negative-stain TEM, SDS-PAGE gel electrophoresis and CD spectroscopy. (A) Electron micrographs of dGAE species at 0 h and 72 h agitation. Scale bar: 500 nm. Middle panel is a higher magnification of the white box in the left panel (scale bar: 200 nm). (B) Non-reducing SDS-PAGE gel of dGAE and dGAE-488 shown as Coomassie stain (left panel) and fluorescence (right panel). Black arrowheads point to monomers and dimers, tetramers and insoluble fibrils in the well. (C) CD spectra of the whole assembly mixture for dGAE and dGAE-488 at 0 h and 72 h agitation.
FIGURE 2 shows Exposure to aggregated dGAE but not soluble (monomer/dimer) dGAE results in increased cell death. 100 µM dGAE was agitated for 72 h to produce fibrils. Soluble (0 h) or aggregated (72 h) species (1 µM) were added to cells and left to incubate for 24 h. (A) Representative widefield images following exposure to buffer or dGAE with ReadyProbes^{®} reagent, showing total nuclei in blue and nuclei of dead cells in green. Scale bar: 100 µm (B) The percentage cell death was quantified for all conditions. Data shown are averages from six fields of view from 4-6 independent experiments ± SEM. A one-way ANOVA shows a significant difference between groups (F = 11.26, R² = 0.12, p < 0.0001). Dunnett's multiple comparisons shows a significant difference between cells treated with buffer only (21.6 ± 1.6%) and cells treated with dGAE fibrils (72 h dGAE) (34.0 ± 2.9%) (p < 0.0001) but not between buffer-treated cells and soluble dGAE-treated cells (24.1 ± 1.2%).

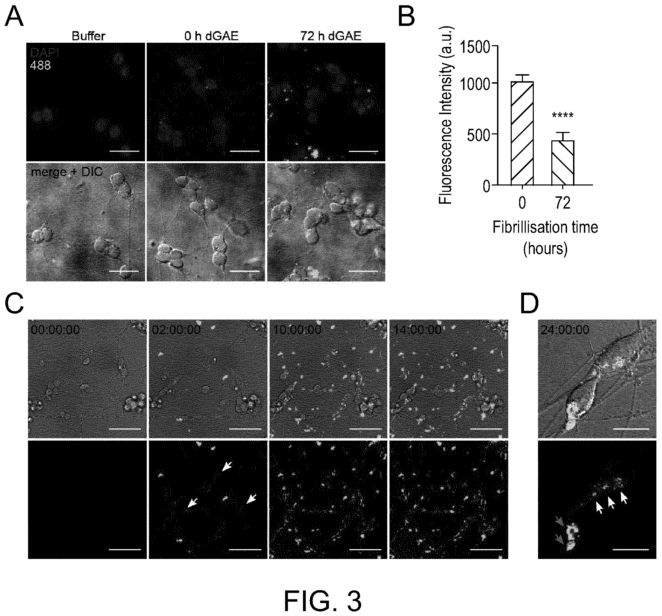

FIGURE 3 shows Soluble dGAE-488 readily internalises into dSH-SY5Y cells. (A) 1 µM soluble dGAE-488 (unagitated) or 72 h agitated (aggregated) dGAE was added to the media of dSH-SY5Y cells and then fixed and visualised by confocal microscopy after 24 h exposure. All scale bars: 20 µm. (B) The 488 fluorescence intensity in the cell body was quantified from the middle of the z-stack from N = 273 cells from 6 independent experiments (0 h) and from N = 120 cells (72 h) from 3 independent experiments. An unpaired t-test with Welch's correction shows a significant difference in 488 fluorescence intensity between 0 h (1114 ± 66.75 AU) and 72 h (474.1 ± 95.97 AU) (t = 5.475, df = 237.7, R² = 0.112, p < 0.0001). Data are shown as mean ± SEM. (C) Internalisation of 5 µM soluble dGAE-488 (unagitated) was monitored live using confocal microscopy from t = 0 h to t = 14 h. 488-positive puncta can be seen internalised into the cell body and neurites from 2 h following the initial addition of dGAE-488 (white arrows). Scale bar: 50 µm. (D) Higher magnification images of internalised soluble dGAE-488 after 24 h exposure show a punctate pattern in the cell body (white arrow) and larger perinuclear accumulation (red arrow). Scale bar: 15 µm. One z-slice is shown from the middle of the cell body for all panels.

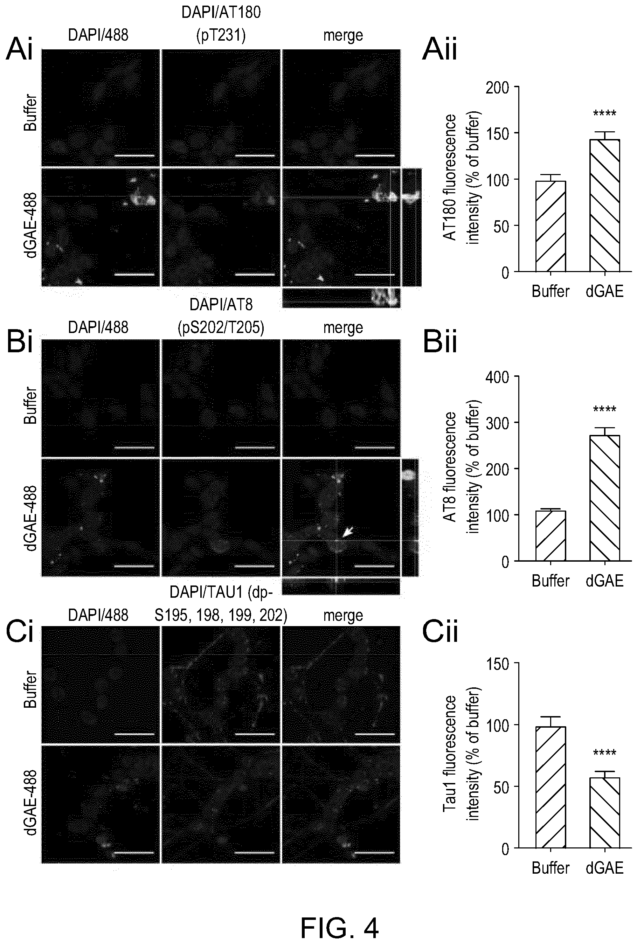

FIGURE 4 shows Exposure to dGAE-488 leads to an accumulation of endogenous phospho-tau in dSH-SY5Y cells. 1 µM soluble dGAE-488 (unagitated) was added to the media of dSH-SY5Y cells for 24 h. Cells were immunolabelled for endogenous tau using p-tau antibodies (Ai) AT180, (Bi) AT8 and (Ci) dephosphorylated Tau-1. One z-slice is shown from the middle of the cell body. Orthogonal views are displayed in Ai and Bi to show potential colocalisation of dGAE-488 with endogenous tau. Scale bar: 20 µm. Each panel shows quantification of fluorescence intensity as a percentage of buffer-treated cells. (Aii) Quantification of AT180 fluorescence (N = 69 cells (buffer), 76 cells (dGAE) from 4 independent experiments). An unpaired t-**test with Welch's correction shows a significant difference in AT180 fluorescence** intensity between dGAE- (143.5 ± 7.8%) and buffer-treated cells (100 t 5.2%) (t = 4.702, df = 128.2, R² = 0.1471, p < 0.0001). (Bii) Quantification of AT8 fluorescence. N = 348 cells (buffer), N = 338 cells (dGAE) from 3 independent experiments. An unpaired t-test with Welch's **correction shows a significant difference in AT8 fluorescence intensity between** dGAE- (270.7 ± 22.5%) and buffer-treated cells (100 ± 3.51%) (t = 7.103, df = 353.4, R² = 0.1249, p < 0.0001). (Cii) Quantification of Tau1 fluorescence. N = 68 cells (buffer), 96 cells (dGAE) from 3 independent experiments. An unpaired t-test with Welch's correction shows a significant difference in Tau-1 fluorescence intensity between dGAE (59.17 ± 3.6%) and buffer-treated cells (100 ± 7.3%) (t = 5.011, df = 99.84, R² = 0.201, p < 0.0001).

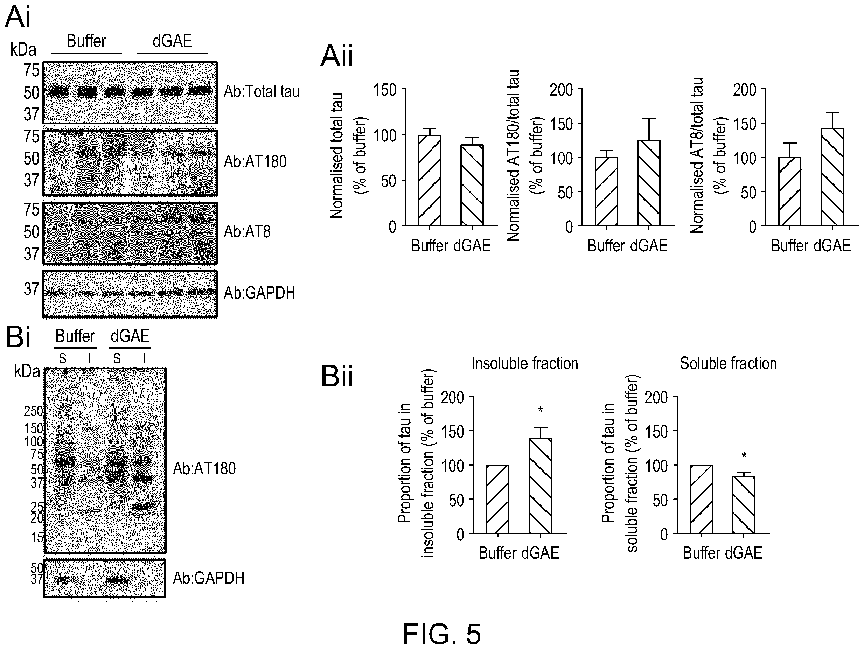

FIGURE 5 shows Exposure to dGAE for 24 h leads to an accumulation of endogenous phospho-tau in the Triton-insoluble fraction of dSH-SY5Y cells. (Ai) Representative western blot of SH-SY5Y cell lysate. 1 µM soluble dGAE (unagitated) was added to the media of dSH-SY5Y cells. After 24 h, cells were lysed in RIPA buffer and run on SDS-PAGE. Blots were probed against total tau, AT180 and AT8. GAPDH was used as a loading control. (Aii) The intensity of bands at 50-70 kDa were quantified for each antibody and normalized against GAPDH. The normalized values for AT180 and AT8 are expressed as a proportion of total tau (percentage of buffer). Data are shown as mean ± SEM from 3 independent experiments. An unpaired t-test shows no significant difference in total tau (p = 0.3101), AT180 (p = 0.4662) or AT8 (p = 0.2119) immunoreactivity between buffer-treated control and dGAE-treated cells. (Bi) Cells were also sequentially lysed in Triton-X 100 lysis buffer and run on SDS-PAGE. S = Triton-X 100 soluble lysate; I = Triton-X 100 insoluble lysate. The A T180- antibody was used to detect endogenous phosphorylated tau and GAPDH was used as a loading control. B) The proportion of tau in the insoluble and soluble fractions was quantified by densitometry and expressed as a percentage of buffer-treated controls. Data are shown as mean ± SEM from 4 independent experiments (1 biological repeat per experiment). An unpaired t-test shows a significant difference in insoluble tau between buffer- (100 ± 0) and dGAE-treated cells (139.4 ± 14.54) (t = 2.709, df = 6, R² = 0.5501, p = 0.0352) and in soluble tau between buffer (100 ± 0) and dGAE-treated cells (82.98 ± 5.48) (t = 3.106, df = 6, R² = 0.6165, p = 0.0201).

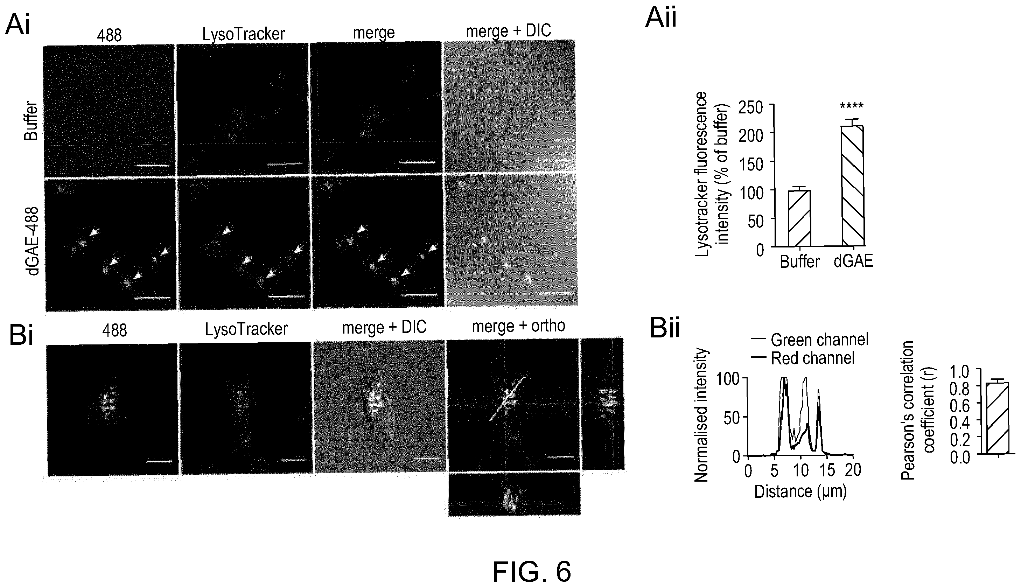

FIGURE 6 shows internalised soluble dGAE-488 is localised to acidic vesicles in dSH-SY5Y cells. Ai) Representative immunofluorescence images of cells exposed to 1 µM soluble dGAE-488 (unagitated) for 24 h. Cells were labelled with LysoTracker^{®} to stain for acidic vesicles including lysosomes and endosomes and were imaged live. dGAE-488 and LysoTracker^{®} labelling is mainly localised in the cell body (white arrows). One z-slice is shown from the middle of the cell body. Scale bar: 50 µm. (Aii) Quantification of LysoTracker^{®} fluorescence intensity as a percentage of buffer-treated cells. Data shows mean ± SEM pooled from 3 independent experiments. N = 170 cells (buffer), N = 146 cells (dGAE). An unpaired t-test with Welch's correction shows a significant difference in LysoTracker^{®} fluorescence intensity between dGAE (211.9 ± 9.89%) and buffer-treated cells (100 ± 5.23%) (t = 10, df = 222.6, R² = 0.3101, p < 0.0001). Bi) Higher magnification of a single cell labelled with LysoTracker^{®} containing internalised dGAE-488. The last panel displays orthogonal views to show colocalisation of dGAE-488 with LysoTracker^{®}. One z-slice is shown from the middle of the cell body. Scale bar: 10 µm. (Bii) Normalised values of fluorescence intensity for 488 (green) and LysoTracker (red) along the region indicated by the white line (20 µm) in the last panel in (Bi). Colocalisation of dGAE-488 and LysoTracker^{®} is confirmed by the **Pearson's correlation coefficient between the green and red channel. The average Pearson's** R value was 0.8382 ± 0.036 (p < 0.0001 for each cell) (N = 7 cells).

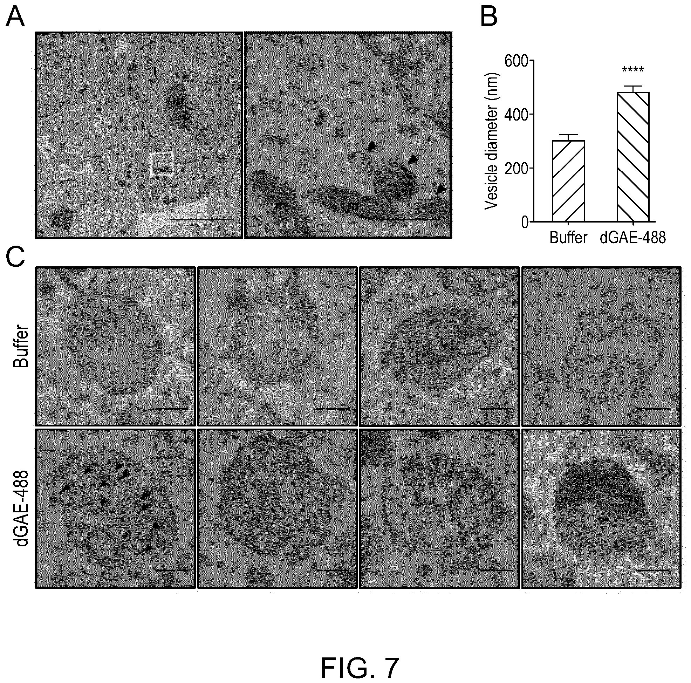

FIGURE 7 shows internalised soluble dGAE-488 is localised to vesicular structures in dSH-SY5Y cells. dSH-SY5Y cells were exposed to 1 µM soluble dGAE-488 (unagitated) for 24 h and were processed for immunogold electron microscopy. Anti-488 antibody was used to label dGAE, detected by 10-nm gold particles. A) Electron micrograph showing preserved ultrastructure of cells, with the main cellular compartments labelled: n, nucleus; nu, nucleolus; m, mitochondria; v, vesicular structure. Black arrowheads point to vesicular structures. The black outlined image represents a magnified image of the area in the white box. Left scale bar: 5 µm. Right scale bar: 1 µm. B) Quantification of vesicle diameter. Data is shown as mean ± SEM pooled from 2 independent experiments. N = 34 vesicles (buffer), N = 45 vesicles (dGAE-488). An unpaired t-test showed a significant difference in vesicle diameter between buffer (303.7 ± 20.03) and dGAE-treated cells (478.4 ± 22.48) (t = 5.601, df = 77, p < 0.0001, R² = 0.2895). C) Higher magnification electron micrographs of vesicular structures from buffer-treated cells and dGAE-488 treated cells. Black arrowheads point to some examples of gold particles. Scale bar: 200 nm.

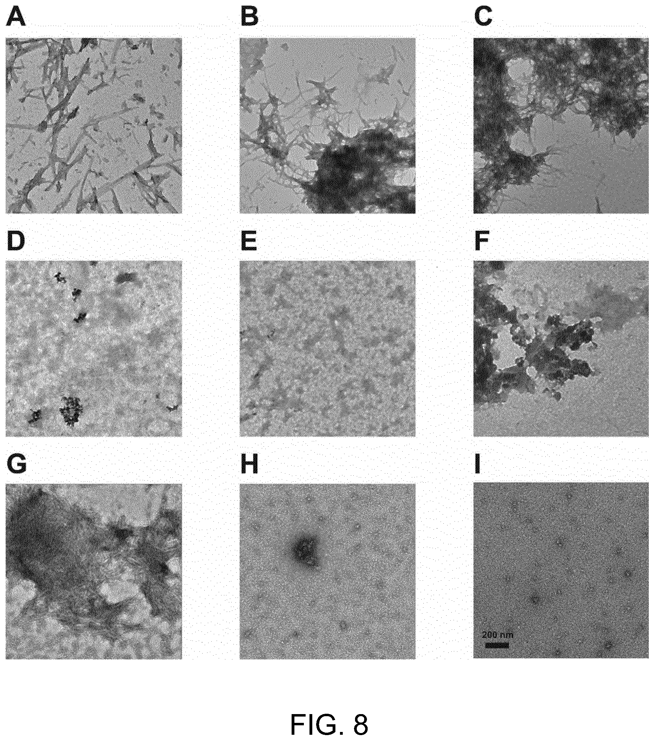

FIGURE 8 shows transmission electron microscopy images of dGAE assembled in the presence and absence of antibodies. (A) dGAE (100 µM); (B) dGAE (25 µM); (C) dGAE (10 µM); (D) dGAE (100 µM) + s1D12 (25 µM; 4:1); (E) dGAE (25 µM) +s1D12 (25 µM; 1:1); (F) dGAE (10 µM) + s1D12 (2.5 µM; 4:1); (G) dGAE (10 µM) + anti-ovalbumin (2.5 µM; 4:1); (H) s1D12 (25 µM); (I) anti-ovalbumin (2.5 µM).Fibrils are only observed in preparations in the absence of s1D12 (A-C) or in a control of dGAE prepared in the presence of a non-tau IgG antibody, anti-ovalbumin (G). When s1D12 is added to the assembly mixture at dGAE protein:antibody ratios of either 1:1 or 4:1 (D, E, and F), no fibril formation is observed. Fibrils are also absent from antibody controls in the absence of dGAE (H and I). Scale bar (on I for all panels), 200 nm.

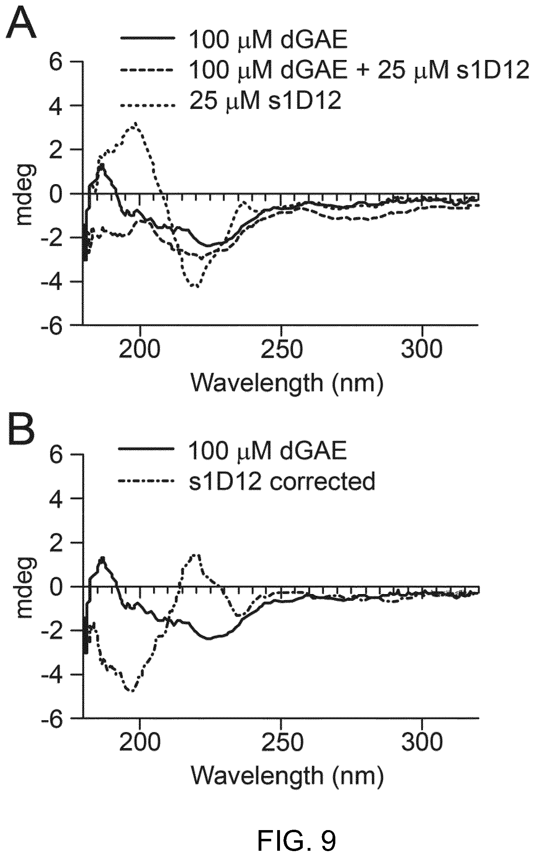

FIGURE 9 shows CD spectra in millidegrees (mdeg) of (A) 100 µM dGAE with (dashed line) and without (solid line) 25 µM s1D12 (ratio 4:1) and antibody alone (dotted line) and (B) with the antibody spectra subtracted. dGAE (solid line) displays a β-sheet conformation (positive ~200 nm, negative ~220 nm) but shows random coil (negative at ~200 nm, positive at ~220 nm) when the s1D12 spectra is subtracted (B; dashed and dotted line), indicating that dGAE has not assembled into fibrils.

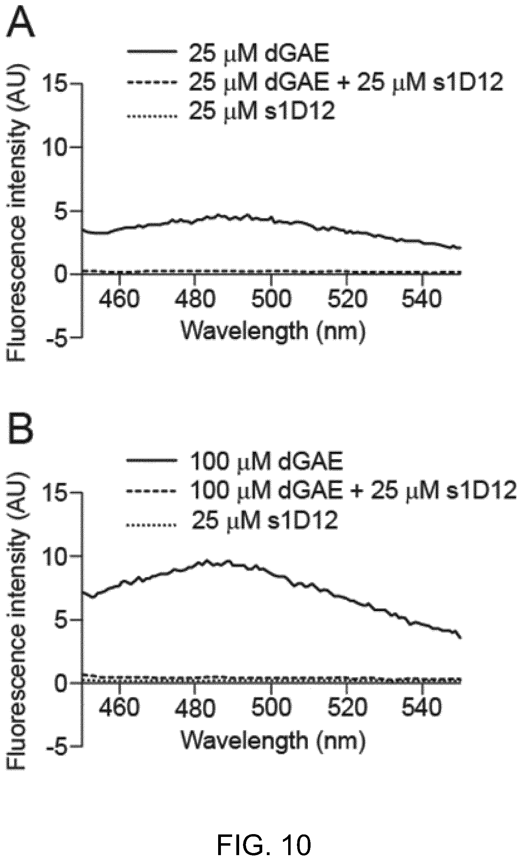

FIGURE 10 shows fluorescence of samples incubated with ThS. An emission peak at 483 nm is clearly observed with 25 (A, 1:1) and 100 µM (B, 4:1) dGAE (solid lines) which is abolished when s1D12 is included in the assembly mixture (dashed lines), showing that only samples that do not include s1D12 contain self-assembled amyloid fibrils. Dotted lines show that s1D12 alone does not contribute to the fluorescence.

### Materials and Methods

### Preparation of recombinant dGAE

Purified recombinant truncated tau (dGAE, corresponding to amino acid residues 297 - 391 using numbering from 2N4R tau) was used throughout the study. Recombinant tau protein 297-391 was purified as previously described in Al-Hilaly etal (J. Mol. Biol., vol. 429, no. 23, pp 3650-3665 (2017)). Following purification, dGAE exists in a predominantly random coil conformation and consists mainly of soluble monomer and dimer as previously characterised and described in Al-Hilaly et al (J. Mol. Biol., vol. 429, no. 23, pp 3650-3665 (2017)).

### Alexa Fluor^{®} 488 labelling of tau protein

To generate fluorescently tagged tau protein (dGAE-488), dGAE was covalently labelled with Alexa Fluor 488^{®} (Life Technologies) by mixing 200 µl protein (425.2 µM) with 10 µl 113 nM Alexa Fluor^{®} TFP ester and 20 µl 1M sodium bicarbonate (pH 8.3). The mixture was left to incubate in the dark for 15 minutes at room temperature. Zeba 7K MWCO columns (Thermo Scientific) were equilibrated by adding 1 ml 10 mM phosphate buffer (pH 7.4) and centrifuging at 1,000 x g for 2 minutes at 4 °C. The eluate was discarded and the process was repeated three times. The protein/dye mixture was added drop-wise onto the top of the column **immediately followed by 40 µl phosphate buffer and was** centrifuged at 1,000 x g for 2 minutes at 4 °C. The protein solution was kept on ice and the absorbance at 280 nm (A₂₈₀) was measured using a NanoDrop spectrophotometer. The protein concentration was calculated using the A₂₈₀, and the molar extinction coefficient of dGAE (1,400 cm⁻¹ M⁻¹), taking into account the absorption of the dye at A₄₉₄. dGAE has 14 lysine residues, which are all potential sites at which the 488 dye can bind. The extent of labelling was determined by the A₄₉₄ and the molar extinction coefficient of the dye (71,000 cm⁻¹ M⁻¹). The protein (dGAE-488) was used immediately for subsequent experiments or subjected to agitation *in vitro.*

### In vitro assembly of dGAE and dGAE-488

*In vitro* assembly of dGAE and dGAE-488 was performed as previously described in Al-Hilaly et al (J. Mol. Biol., vol. 429, no. 23, pp 3650-3665 (2017))**without reducing agent. Briefly, 100 µM** protein was diluted in 10 mM phosphate buffer (pH 7.4) and incubated at 37 °C whilst agitating with a speed of 700 rpm on an Eppendorf ThermoMixer^{®} for 72 hours. Samples were visualised using negative stain transmission electron microscopy (TEM). All experiments were conducted using the stock 100 **µM dGAE in phosphate buffer.**

### Negative stain TEM

**Aliquots (4 µl) of dGAE assembly mixtures (100 µM in phosphate buffer, pH 7.4) were placed on 400-**mesh carbon-coated grid and incubated for 1 minute. After removing excess solution with filter paper, **the grid was washed with 4 µl filtered Milli**-Q water for 1 minute and blotted. The grids were negatively **stained with 4 µl filtered 2 % (w/v) uranyl acetate for 1 minute, blotted dry and left to air dry for at** least five minutes. Grids were examined on a JEOL JEM1400-Plus Transmission Electron Microscope at 100 kV and electron micrograph images were collected using 4k x 4k One View (Gatan) camera.

### Circular dichroism spectroscopy

Circular dichroism spectroscopy (CD) was performed using a Jasco Spectrometer J715 and spectra collected in triplicate at a maintained temperature of 21 °C. Protein samples were placed into 0.02 mm path length quartz cuvettes (Hellma) and scanned from 180 to 320 nm. CD data were converted to molar ellipticity (deg·cm²·dmol⁻¹).

### Cell culture and dGAE treatment

Undifferentiated SH-**SY5Y human neuroblastoma cells were grown in Dulbecco's Modified Eagle** Medium/Nutrient Mixture F-12 (DMEM/F-12) supplemented with 10% Foetal Calf Serum (FCS), 1% Penicillin/Streptomycin (P/S) and 1% L-glutamine. For differentiation, SH-SY5Y cells were plated at a density of 50,000 cells per well in 24-well plates or 300,000 cells per well in 6-well plates. For immunofluorescence, cells were plated onto Menzel-Gläser coverslips (Thermo Scientific). For live-cell imaging, cells were plated on 35-mm dishes on a 1.5-mm coverslip (Mattek). On the first day of differentiation, media was replaced with low-serum culture media (DMEM/F-12 containing 1% FCS, 1% P/S and 1% L-**glutamine) containing 10 µM** *all-trans* retinoic acid (RA) (Sigma-Aldrich) and cells were incubated for 48 h. On day 3, this process was repeated with fresh RA and cells were incubated for a further 48 h. On day 5, the cells were washed once in serum-free culture media to remove traces of serum. Serum-free culture media (DMEM/F-12 containing 1% P/S and 1% L-glutamine) containing 50 ng/ml brain-derived neurotrophic factor (STEMCELL Technologies) was added to the cells and incubated for 48 h. Differentiated cells (dSH-SY5Y) were ready to use for experiments on **day 7. For dGAE treatment, cells were exposed to** 1 **µM dGAE (either soluble without agitation or** after agitation) and incubated for 24 h. All experiments were conducted using dSH-SY5Y cells.

### Cell viability assay

Following the addition of dGAE, cell viability was measured using ReadyProbes^{®} Cell Viability Imaging Kit (Life Technologies). The kit contains a NucBlue^{®} reagent to label all cells (blue) and a NucGreen^{®} reagent to label dead cells only (green). For tagged dGAE, NucRed^{®} reagent was used **to label dead cells (red). One drop of each reagent was added to cells** in 500 µl media as described in the manufacturers protocol (Life Technologies). Cells were incubated with the reagents at 37 °C for 15 minutes and the media was replaced with Live Cell Imaging Solution (Invitrogen^{™}, Thermo Scientific). Cells were imaged using a Zeiss Cell Observer Axiovert 200M microscope. DAPI fluorescence was captured using a G 365 excitation filter and a LP 420 emission filter with a FT 395 dichroic. Green fluorescence was captured using a FITC filter set (BP 450-490 excitation filter, BP 515-565 emission filter and FT 510 dichroic). Identical acquisition settings were used for all replicates and images were analysed using FIJI. Six fields of view were taken per sample and an average of 4500 cells were analysed per condition. The proportion of buffer-treated cell death was quantified by converting the images to grayscale followed by manually adjusting the threshold and converting it into a binary image to highlight live DAPI-stained cells. The number of cells was automatically counted. The dead cells were counted in the same way and cell death was expressed as a percentage of buffer-treated cells.

### Cell lysis and fractionation

Cells were detached from the coverslip by incubation in 0.25 % trypsin-EDTA (Gibco^{™}) and then mixed with 5 ml culture media. The cells were harvested by centrifugation at 500 × g for 5 minutes and the supernatant was discarded. The cell pellet was resuspended in ice-cold PBS and centrifuged at 500 × g at 4 °C. Cells were lysed in 1 % Triton lysis buffer (1% Triton X-100 (v/v), 150 mM NaCl and 50 mM Tris-HCl, **pH 7.6) containing Halt^{™} protease inhibitors (Thermo Scientific) and** phosphatase inhibitors (Thermo Scientific) for 15 minutes on ice. The samples were centrifuged at 16,000 × g for 30 minutes at 4 °C, and the supernatant was collected (Triton-soluble fraction). The pellet was resuspended in SDS lysis buffer (1% SDS (w/v), 150 mM NaCl and 50 mM Tris-HCl, pH 7.6) containing protease and phosphatase inhibitors. The samples were centrifuged at 16,000 × g for 30 minutes at room temperature and the supernatant was collected (Triton-insoluble fraction). Protein concentration in the Triton-soluble fractions were **determined using the Pierce^{™} BCA Protein Assay** Kit (Thermo Scientific) according to the manufacturer's instructions.

### SDS-PAGE and Western blotting

For cell lysates, Triton-soluble protein (20 µg) and an equal volume of Triton-insoluble protein were each mixed with Laemmli sample buffer (4 x) (Bio-Rad Laboratories) containing 5 % (v/v) β-mercaptoethanol and heated at 95 °C for 5 minutes. For recombinant protein, 3 µg was mixed with sample buffer without reducing agent or boiling. All samples were centrifuged for five minutes at 1000 × g and samples were loaded onto 4-20 % Mini-PROTEAN^{®} precast gels (Bio-Rad Laboratories) and run at 120 V for 1 h in Tris-glycine running buffer (25 mM Tris, 192 mM glycine, pH 8.3), or until the sample buffer reached the end of the gel. For Coomassie staining, the gel was washed three times in double distilled water for five minutes and stained with Imperial^{™} protein stain (Thermo Scientific) for 1 h and then destained overnight in double distilled water. The stained gel was scanned using an HP Photosmart C5280 scanner. For western blotting, the separated proteins on the gel were transferred to nitrocellulose membrane (0.45 µm) at 200 mA for 90 minutes. The membranes were blocked in 5 % (w/v) bovine serum albumin (BSA) in Tris-buffered saline (50 mM Tris-HCl, pH7.4, 150mM NaCl) containing 0.1 % (v/v) Tween-20 (TBS-T) for 1 h rocking at room temperature. Membranes were incubated with primary antibodies diluted in 5 % BSA in TBS-T overnight at 4 °C. The following primary antibodies and dilutions were used; Anti-Tau (polyclonal, total tau) (Thermo Scientific) (1:2500), AT180 (anti-pT231) (Thermo Scientific) (1:1000), AT8 (anti-pS202-T205) (1:1000) (Thermo Scientific), anti-GAPDH (1:5000) (Abcam). The next day, membranes were incubated in horseradish peroxidase (HRP)-anti-mouse antibody (1:5000) (Sigma-Aldrich) or HRP-anti-rabbit antibody (1:5000) (Abcam) in 5 % BSA in TBS-T for 1 h at room temperature. The membranes were washed 3 × 10 mins in TBS- T between antibody incubations. Immunoreactive protein bands were detected using ECL substrate (Clarity^{™}, Bio-Rad) and X-ray films were scanned using an HP Photosmart C5280 scanner. FIJI was used to quantify the bands in arbitrary densitometry units. The density of bands corresponding to full-length tau (50-70 kDa) were determined and normalised against amount of GAPDH. These values were used to calculate the proportion of total tau that is phosphorylated, expressing the proportions as a percentage of the buffer-treated control group. For the quantification of Triton-insoluble and soluble protein, the proportion of tau in the insoluble and soluble fraction was calculated using the equations *insoluble*/*(soluble* + *insoluble)* or *soluble*/*(soluble* + *insoluble),* respectively and expressed as a percentage of the respective soluble or insoluble control value.

### Immunofluorescence

Cell culture medium was aspirated from dSH-SY5Y cells and washed once with PBS. Cells were fixed in 4 % (w/v) paraformaldehyde (PFA) in PBS for 15 minutes followed by three washes in PBS. For permeabilization, the cells were incubated in 0.25 % (v/v) Triton X-100 in PBS for 10 minutes. Cells were blocked in 2 % (w/v) BSA in PBS for 1 h, followed by three washes in PBS. Primary antibodies diluted in 2 % (w/v) BSA in PBS and were incubated with the cells for 1 h. The primary antibodies and dilutions used were; AT180 (anti-pT231) (1:250) (Thermo Scientific), AT8 (anti-pS202-T205) (1:500) (Thermo Scientific) and Tau1 (dephosphorylated tau at S195, 198, 199 and 202) (Merck Millipore). Cells were incubated with goat anti-mouse-Alexa Fluor^{®} 594 (1:1000) (Invitrogen^{™}, Thermo Scientific) diluted in 2 % (w/v) BSA in PBS for 1 h in the dark. Cells were washed 3 times in PBS between antibody incubations. Cells were mounted onto glass slides using Prolong Gold mounting medium containing 4,6-diamidino-2-phenylindole (DAPI). Mounted slides were stored in the dark at room temperature for 24 - 48 h before imaging and kept at 4 °C for long term storage. Cells were imaged using Leica SP8 confocal microscope.

### Labelling of acidic organelles

Cells were plated on 35-mm dishes on a 1.5-mm coverslip (Mattek). Following differentiation, dGAE-488 (5 µM) was added to the cells for 24 h. For labelling lysosomes and endosomes, LysoTracker^{®} red (Life Technologies) was diluted in culture media at a final concentration of 50 nM and incubated for 90 mins before imaging using a Leica SP8 confocal microscope.

### Confocal microscopy

All confocal microscopy imaging was carried using a Leica SP8 confocal microscope. The instrument setting used PMT 3 and PMT Trans channels/lasers and images were acquired with a HC PLAPoCs2 63 x/1.40 oil-immersion objective lens. Samples were scanned sequentially to prevent spectral bleed through. All images were collected as Z-stacks for all channels using a step size of 0.5 µm. Five to ten Z-stacks were taken for each sample and each experiment was repeated three times or more. To monitor live uptake of dGAE-488, the environment was maintained at 37 °C with humidified CO₂ and the Adaptive Focus Control feature was used to maintain constant focal planes throughout the course of the experiment.

### Processing cells for TEM

DSH-SY5Y cells were treated with Alexa Fluor^{®} **488 containing buffer, or 10 µM freshly prepared** dGAE-488 for 24 h. The cells were washed once in PBS, scraped into a tube and centrifuged at 500 × g for 5 minutes. The media was removed and the cells were suspended in a 1:1 mixture of prewarmed culture media: **4** % (v/v) PFA for 15 minutes at 37 °C. The cells were centrifuged at 500 × g for 5 mins and the cells were resuspended in fresh 4 % (v/v) PFA and 0.1 % (v/v) glutaraldehyde (GA) in 0.1 M phosphate buffer (pH 7.4) for 3 h at room temperature. The fixed cells were centrifuged at 1,000 × g for 5 minutes. The supernatant was discarded and the pellet was suspended in 50 mM glycine in PBS for 10 minutes at room temperature. The cells were centrifuged at 1,000 × g for 5 minutes and the pellet was **washed three times with 0.1 M cacodylate buffer (pH 7.4). Around 200 µl** 4 % (w/v) low melting point agarose was added to the cells and immediately centrifuged at 1,000 × g for 10 minutes at 30 °C. The tube was immediately transferred to 4 °C or on ice for 20 minutes to solidify the agarose. The agarose-embedded cell pellet was transferred to a new tube and washed 2-3 times with 0.1 M cacodylate buffer (pH 7.4). The pellet was post-fixed in a reduced osmium solution (1 % (v/v) osmium tetroxide, 1.5 % (w/v) potassium ferrocyanide in 0.1 M cacodylate buffer, pH 7.4) for 1 h at 4 °C followed by washing three times in 0.1 M cacodylate buffer (pH 7.4) and three times in double-distilled H2O for 5 minutes each. The pellet was dehydrated in an ethanol series consisting of 30, 50, 75, 90 and 95 % ethanol for 15 minutes each at 4 °C followed by three incubations in 100 % ethanol for 20 minutes each at 4 °C. The sample was then infiltrated in a 2:1 mixture of 100 % ethanol:Unicryl^{™} resin (for 30 minutes followed by a 1:2 mixture of 100 % ethanol:Unicryl^{™} resin (BBI Solutions) for 30 mins. Finally, the pellet was transferred to a BEEM capsule (Agar Scientific) and infiltrated in complete Unicryl^{™} resin overnight at 4 °C. The resin was cured using light- polymerisation for 48 h by illumination from the underside of the BEEM capsules from a 12 V, 100 W type 6834 Philips projection lamp at a distance of 35 cm. Ultrathin sections (70 nm) were taken using a diamond knife on a Leica EM UC7 ultramicrotome fitted with a Leica M80 microscope (Leica Microsystems) and placed on 300 Mesh Hexagon Nickel 3.05 mm grids (Agar Scientific Ltd) before proceeding with immunogold labelling.

### Immunogold labelling TEM

**A modified PBS (pH 8.2) containing 1% BSA, 500 µl/L Tween-20,** 10 mM Na- EDTA and 0.2 g/L NaN₃ (termed PBS+) was used throughout the following procedures for all dilutions of antibodies and gold probes. The ultrathin sections were initially blocked using normal goat serum (diluted 1:10 dilution in PBS+; Sigma-Aldrich) for 30 minutes at room temperature and then incubated with anti-Alexa Fluor^{®} 488 primary antibody (1:50) (Thermo Scientific). The sections were washed three times in PBS+ for 2 minutes each followed by incubation with 10 nm gold particle-conjugated goat anti-rabbit IgG secondary probe at a 1:10 dilution in PBS+ for 1 h at room temperature. The sections were washed three times in PBS + for 10 minutes each and four times in distilled water for 5 minutes each. Immunogold-labelled thin sections were subsequently post-stained in 2 % (w/v) uranyl acetate for 1 h before imaging on the TEM.

### image analysis

FIJI (https://fiji.sc) imaging processing package (J. Schindelin et al., Nat. Methods, vol. 9, no. 7, pp. 676-682, (2012)) was used for all image analysis. For quantification of fluorescence intensities, images were Z-projected to maximal intensity. Five to ten fields of view were taken from each condition and an average of 50 cells per condition were subjected to analysis. Firstly, a region of interest (the cell body) was drawn around an individual cell, excluding cells that had fused nuclei. Area-integrated intensity and mean grey value were measured as well as three selections from around the cell with no fluorescence (background). The corrected total cell fluorescence (CTCF) was then calculated using the equation: CTCF=Integrated Density - (Area of selected cell x Mean fluorescence of background readings)

For quantification of internalised dGAE-488, a focal plane from the middle of the cell that contained maximal DAPI fluorescence was selected and subjected to analysis.

### Data analysis

Data and statistical analyses were performed using Microsoft Excel and GraphPad Prism 7. All data are expressed as the mean ± standard error of the mean (SEM). When comparing two groups, **Student's unpaired t-test with Welch's correction was used to determine statistical significance. When** comparing more than two groups, one**-way analysis of variance (ANOVA) with Dunnet's post**-hoc test was used to determine differences between experimental groups and a control group. Differences were considered to be statistically significant if *p* < 0.05.

### Example 1: Examination of formation of fibrils by dGAE and dGAE-488

Soluble dGAE was fluorescently labelled using an Alexa-fluor 488 tag (dGAE-488) to enable internalisation of dGAE assemblies to be monitored and to distinguish exogenous dGAE from endogenously expressed tau. On average, there were 0.1 moles of 488 dye per mole of protein after every labelling reaction. The Alexafluor-tag labels amino groups at the N-terminus and on lysine residues, and hence we examined the tagged and untagged dGAE using TEM, SDS-PAGE and CD spectroscopy to determine whetherthe label affects aggregation and/or filament formation. Using the non-reducing conditions for aggregation established previously (Al-Hilaly et al., J. Mol. Biol., vol. 429, no. 23, pp 3650-3665 (2017)), dGAE and dGAE-488 produced morphologically similar short twisted fibrils (figure 1A). SDS-PAGE of dGAE and dGAE-488 showed the presence of both the 10/20-kDa and of the 12/24-kDa forms (monomer/dimer), with the latter predominating in the non-agitated dGAE preparation at 0 h. We have previously shown that dGAE is random coil at 0h and consists mainly of SDS soluble monomer and dimer, although the solution likely contains a mixture of low molecular weight species. Over time, the dGAE self-assembles to form β-sheet rich filaments (Al-Hilaly et al.,I J. Mol. Biol., vol. 429, no. 23, pp 3650-3665 (2017)). The dGAE-488 preparation showed slightly increased intensity of dimer bands at 0 h. Electron micrographs of the proteins at 0 h and 72 h show similar size species in both the dGAE and dGAE-488 preparations (figure S1), with round species at 0 h ranging from 10-80 nm in diameter (figure S1Aii) and fibrils at 72 h ranging from 20-350 nm in length (figure S1Bii). After fibrillisation induced by agitation for 72 h, there was less of the 12-kDa monomeric form for dGAE compared with dGAE-488, and more of both preparations were retained in the gel well (Al-Hilaly etal., J. Mol. Biol., vol. 429, no. 23, pp 3650-3665 (2017)). CD spectra were similar for both preparations, with similar intensity minima at 198 nm (predominantly random coil conformation) at 0 h and the expected decrease in random coil signal by 72 h, which accompanied an increase in insoluble β-sheet structures with a minimum around 218 nm. We have previously demonstrated the β-sheet signal at 218nm in the pellet following centrifugation to separate it from supernatant (Al-Hilaly et al., J. Mol. Biol., vol. 429, no. 23, pp 3650-3665 (2017)). The results presented show that dGAE and dGAE-488 form structurally and morphologically similar fibrils.

### Example 2: Study on induction of cell death by dGAE

**Aggregated dGAE (100 µM agitated for 72 h) and soluble dGAE (100 µM 0 h, no agitation) were** applied **at a concentration of 1 µM directly to dSH**-SY5Y cells and incubated for 24 h. Cell viability was measured after 24 h using the ReadyProbes^{®} assay to measure cell death (figure 2A). Although there was some increase in the percentage of dead cells due to soluble dGAE compared with buffer treatment, this did not reach statistical significance (figure 2B). There was a significant increase in cell death following incubation with aggregated dGAE (34 ± 2.9%, p < 0.0001) compared with buffer only control (21.6 ± 1.6%). **Increasing the concentration of soluble dGAE up to 20 µM produced no** further increase in cell death after incubation for 24 h (figure S2). dGAE and dGAE-488 showed **comparable effects on cell death following the incubation of cells with 1 µM soluble** or aggregated protein, although the soluble form of dGAE-488 was marginally more toxic, but with no difference after 72h fibrillisation. The results show that extracellularly-applied aggregated dGAE but not soluble dGAE induces acute cell death.

### Example 3: Internalisation of dGAE-488

The uptake of soluble and aggregated dGAE into dSH-SY5Y cells was investigated in order to examine whether aggregation state affects the efficiency of internalisation. The labelled dGAE-488 form was used to permit uptake to be visualised and measured having corrected for total cell fluorescence. Soluble or aggregated (72h) dGAE-488 was incubated with dSH-SY5Y cells at 1 µM for 24 h. Confocal microscopy analysis showed that following exposure to soluble dGAE-488, fluorescence was observed within the cells as punctate staining (figure 3A). Aggregated dGAE-488 was internalised significantly less efficiently than the soluble form (474 ± 96 versus 1,114 ± 67 AU per cell, *p* < 0.0001; figure 3B), and larger accumulations of dGAE-488 fluorescence could be observed outside the cells. Live cell imaging was used to monitor the internalisation of soluble dGAE-488. Some dGAE-488 remains outside cells and increases in brightness and size over time, consistent with some continued assembly in the medium. Internalised dGAE-488 could be detected within neurons after 2 hours of incubation and uptake was found to increase over time (figure 3C). By 24 h, both distinct punctate staining pattern as well as larger perinuclear accumulations were observed (figures 3C & 3D). Although the time-intervals of the live-cell imaging did not allow for detailed examination of intracellular transport, the internalised dGAE-488 was observed being trafficked along the processes. The results show labelled dGAE-488 is internalised by dSH-SY5Y cells.

### Example 4: Phosphorylation changes following dGAE internalisation

The results presented herein suggest that incubation with aggregated dGAE leads to significant cell death after incubation for only 24h, whereas soluble dGAE is internalised but does not appear to be significantly toxic following 24h incubation. Therefore, it was further investigated whether internalised soluble dGAE-488 may be capable of altering the aggregation or phosphorylation state of **endogenous tau. Following exposure to soluble** 1 **µM dGAE-488,** cells were fixed and immunolabelled using antibodies recognising tau epitopes outside of the dGAE sequence allowing specific detection of only endogenous tau (figure 4A-C). Cells were examined for colocalisation of internalised dGAE-488 and endogenous tau and the fluorescence intensity of labelled tau in the cell body was quantified in individual cells for each antibody. Labelling with AT180 (tau phosphorylated at T231; pT231) and AT8 (tau phosphorylated at 202-205; pS202-pT205) was largely absent in buffer-treated cells but increased significantly following incubation with soluble dGAE-488, with some cells showing colocalisation of dGAE-488 with phosphorylated endogenous tau (figure 4A, B). We have previously shown that tau dephosphorylated between amino acids 192-204 detected using Tau-1 antibody, is found in the nucleus in dSH-SY5Y cells (Maina et al., Acta Neuropath. Comm, Vol. 6 No. 70, pp 1-13 (2018)). Here, we identified tau-1 labelling in the nucleus as expected but found that there was no colocalisation with dGAE-488. Quantification of Tau-1 fluorescence showed a significant decrease in fluorescence intensity in cells treated with dGAE-488 (figure 4C) which may indicate that the tau becomes phosphorylated. The results show internalised dGAE-488 leads to increased phosphorylation of endogenous tau.

### Example 5: Effects of exposure of cells to soluble dGAE-488

Western blots of the whole cell lysates were performed to examine the effect of exogenously applied dGAE on endogenous tau phospho-epitopes. Comparison of cells incubated with soluble dGAE with buffer-treated control cells revealed no change in levels of total tau and marginal increases in AT8 or AT180 immunoreactivity that did not reach statistical significance. While western blots measure total levels of phospho-tau in cells, immunofluorescent imaging is able to highlight subcellular localisation of any increases. In order to determine the effects of internalised soluble dGAE-488 on the solubility of endogenous tau in neurons, we undertook sequential extraction of tau protein from cell lysates following solubilisation with 1% Triton X-100 and centrifugation (16,000g for 30 minutes). Following incubation with soluble dGAE for 24 h, there was a clear redistribution of tau protein immunolabelled with AT180 from Triton-X 100-soluble to Triton-X 100-insoluble fractions following incubation with soluble dGAE compared with buffer-treated controls. dGAE incubation produced a reduction in soluble tau immunoreactive with AT180 (83.0 ± 5.5%, *p* = 0.0201) and an increase in the insoluble form (139.4 ±14.5%, *p* = 0.0352) (figure 5A, B). Unexpectedly, a small amount of tau was observed in the insoluble fraction of the buffer treated control but the difference between control and treated cells was significant. Furthermore, a new truncated tau species having gel mobility of 20-25-kDa with epitopes located in the N-terminal half of the tau molecule appeared in the insoluble fraction. This was more intense in the cells incubated with dGAE than with buffer alone. The results show that exposure of cells to soluble dGAE-488 leads to an increase in triton-insoluble endogenous tau.

### Example 6: Localisation of internalised dGAE-488

Intracellular fluorescent punctate particles were observed within the cytoplasm, close to the nucleus after 24 h of exposure of neurons to soluble dGAE-488. This staining pattern was consistent with its localisation in cytosolic vesicles. We therefore examined whether internalised dGAE-488 was localised to endosomal/lysosomal compartments. Soluble dGAE-488 was incubated with cells for 24 h and stained with LysoTracker^{®}, a dye that labels acidic organelles (lysosomes and late endosomes), for 30 mins and then fixed and visualised by confocal microscopy. There was a significant increase in the intensity of the LysoTracker^{®} fluorescence in cells exposed to soluble dGAE-488 compared to buffer-treated control cells (211.9 ± 9.9%, *p* < 0.0001; figure 6Ai, Aii). Internalised dGAE-488 showed a strong colocalisation with LysoTracker^{®} following quantification in **Z-stacks using the Pearson's correlation coefficient** (0.8382 ± 0.036, *p* < 0.0001; figure 6Bi, Bii). The results show that internalised dGAE-488 is localised to perinuclear acidic vesicles.

### Example 7: Ultrastructure of accumulated tau and dGAE-488 in cells

TEM was used to examine the ultrastructure of accumulated tau and dGAE-488 in cells. dSH-SY5Y were exposed to 10 µM soluble dGAE-488 for 24 h and were processed for immunogold TEM using an antibody against Alexa Fluor^{®} 488 to label dGAE-488 specifically. Vesicular structures and mitochondria were observed within the sectioned cells confirming that the TEM protocol preserved the cellular structure (figure 7A). Although no clear fibrillar structures labelled with anti-488 in the cytoplasm, examination at higher magnifications revealed the presence of particles densely labelled with anti-488 within membrane-bound vesicular structures suggesting an accumulation of 488-labelled protein in these organelles (figure 7C). Similar vesicular structures were also present in vehicle-treated cells but lacked gold labelling. Analysis to compare diameters of the membrane-bound compartments that showed neurons treated with dGAE-488 cells contained vesicles of larger diameter than buffer-treated control cells (448.4 ± 22.5 nm versus 303.7 ± 20.0 nm, p < 0.0001), with the largest vesicles being 700-800 nm and smallest being 200-300 nm (figure 7B). The results show that internalised dGAE-488 is packaged in vesicular compartments.

### Example 8: Testing of agents for effects on action of aggregates of tau protein in neuronal cells

SHSY5Y cells are plated in 24 well plates (30,000 cells per well) and either differentiated using retinoic acid in depleted serum medium, as described previously, or left undifferentiated. Cells are treated in one of the following ways; either (1) pre-incubated in the presence of an agent of interest prior to adding tau aggregates or (2) co-incubated with an agent of interest and tau aggregates administered at the same time. Times for incubation are 24 hours at each stage meaning the total time for (1) is 48 hours and 24 hours for (2). dGAE-488 (1 µM) incubated with cells for 24 hours is sufficient for internalised protein to be seen. Following incubation, cells are photographed and analysis of images performed using imaged to quantify the fluorescence intensity within the cell body to provide a measure of dGAE-488 internalisation. The number of cells is counted to express the level of internalisation as mean fluorescence intensity/cell to take into account different numbers of cells in fields of view. Fragments of dGAE-488 are added at 1 µM. Agents for screening can be tested initially at a single concentration of 10 µM; those showing inhibitory activity are then tested over at a range of concentrations from 2 µM down to 20 nM and the activity of compounds reported as the concentration at which 50% inhibition of activity is observed. The activities to be measured include: (i) internalisation of tau measured by intracellular fluorescence; (ii) intracellular aggregation of tau measured by harvesting cells, lysing them and separating aggregated tau by ultracentrifugation and measuring aggregated tau by immunoblot with anti-tau antibody; and (iii) cytotoxicity measured using the ReadyProbes^{®} assay to measure cell death (figure 2A). The activities are all normalised to the results obtained for cells treated without agent or with treatment vehicle. Compounds exhibiting 50% inhibition with agent at 2 µM or less being considered active and relative activity compared for different agents.

### Example 9: Tau aggregation inhibition assays in vitro

dGAE protein was diluted into 10 mM phosphate buffer, pH 7.4 (PB) with monoclonal antibody s1 D12 at a ratio of 4:1 protein:Ab (100 µM dGAE + 25 µM s1D12 and 10 µM dGAE + 2.5 µM s1D12) or 1:1 (25 µM dGAE + 25 µM s1D12).

"s1D12" refers to a monoclonal antibody disclosed in UK application no. GB2010652.2 filed on 10 July 2020, and an international (PCT) application filed on 9 July 2021 in the name of WisTa Laboratories Ltd. The epitope bound by the **CDRs of the specific binding molecule referred to as "S1D12" herein may be within an** amino acid sequence comprising residues 337 to 349 of SEQ ID NO: 1 or preferably within an amino acid sequence comprising residues 341 to 353 of SEQ ID NO: 1. The epitope may comprise the amino acid sequence of SEQ ID NO: 8 (VEVKSEKLDFKDR). S1D12 comprises the CDRs VHCDR1, VHCDR2, VHCDR3, VLCDR1, VLCDR2 and VLCDR3, wherein each of said CDRs comprises an amino acid sequence as follows:
VHCDR1 comprises the sequence set forth in SEQ ID NO: 9 (NNAVG);
VHCDR2 comprises the sequence set forth in SEQ ID NO: 10 (GCSSDGTCYYNSALKS);
VHCDR3 comprises the sequence set forth in SEQ ID NO: 11 (GHYSIYGYDYLGTIDY);
VLCDR1 comprises the sequence set forth in SEQ ID NO: 12 (SGSSSNVGGGNSVG);
VLCDR2 comprises the sequence set forth in SEQ ID NO: 13 (DTNSRPS); and
VLCDR3 comprises the sequence set forth in SEQ ID NO: 14 (VTGDSTTHDDL).

s1D12 may comprise:
(a) A VH domain comprising the sequence set forth in SEQ ID NO: 15 (QVQLQESGPSLVKPSQTLSLTCTVSGFSLNNNAVGWVRQAPGKVPESLVGCSSDGTCY YNSALKSRLDITRDTSKNQISLSLSSVTTDDAAVYYCTRGHYSIYGYDYLGTIDYWGPGLL VTVSS); and/or
(b) a VL domain comprising the sequence set forth in SEQ ID NO: 16 (QAVLTQPSSVSGSLGQRVSITCSGSSSNVGGGNSVGWYQHLPGSGLKTIIYDTNSRPSG VPDRFSGSRSGNTATLTINSLQAEDEGDYYCVTGDSTTHDDLVGSGTRLTVLG);
or a humanized variant of either thereof.

s1D12 may comprise:
(a) A heavy chain comprising the sequence set forth in SEQ ID NO: 17 (QVQLQESGPSLVKPSQTLSLTCTVSGFSLNNNAVGWVRQAPGKVPESLVGCSSDGTCY YNSALKSRLDITRDTSKNQISLSLSSVTTDDAAVYYCTRGHYSIYGYDYLGTIDYWGPGLL VTVSSAKTTAPSVYPLAPVCGDTTGSSVTLGCLVKGYFPEPVTLTWNSGSLSSGVHTFPA VLQSDLYTLSSSVTVTSSTWPSQSITCNVAHPASSTKVDKKIEPRGPTIKPCPPCKCPAPN LLGGPSVFIFPPKIKDVLMISLSPIVTCVVVDVSEDDPDVQISWFVNNVEVHTAQTQTHRED YNSTLRVVSALPIQHQDWMSGKEFKCKVNNKDLPAPIERTISKPKGSVRAPQVYVLPPPE EEMTKKQVTLTCMVTDFMPEDIYVEWTNNGKTELNYKNTEPVLDSDGSYFMYSKLRVEK KNWVERNSYSCSVVHEGLHNHHTTKSFSRTPGK); and/or
(b) a light chain comprising the sequence set forth in SEQ ID NO: 18 (QAVLTQPSSVSGSLGQRVSITCSGSSSNVGGGNSVGWYQHLPGSGLKTIIYDTNSRPSG VPDRFSGSRSGNTATLTINSLQAEDEGDYYCVTGDSTTHDDLVGSGTRLTVLGGQPKSS PSVTLFPPSSEELETNKATLVCTITDFYPGVVTVDWKVDGTPVTQGMETTQPSKQSNNKY MASSYLTLTARAWERHSSYSCQVTHEGHTVEKSLSRADCS);
or a humanized variant of either thereof.

As a positive control, dGAE was prepared at a final concentration of 10, 25 or 100 µM in 10 mM PB. Negative controls consisted of dGAE with a non-tau IgG antibody at a ratio of 4:1 (10 µM dGAE + 2.5 µM anti-ovalbumin), or antibody alone (25 or 2.5 µM s1 D12 and 2.5 µM anti-ovalbumin). Samples were agitated at 700 rpm at 37 °C for 3 days.

Transmission electron microscopy (TEM), circular dichroism (CD) and Thioflavin S (ThS) assays were performed as detailed in Al-Hilaly et. al. (2018) J. Mol. Biol. 430, 4119-4131. Briefly, TEM grids were prepared by adding 4 µL of sample to a carbon-coated grid followed by a wash with milli-Q filtered water then staining twice with 2% uranyl acetate. Grids were air dried and then imaged using a JEOL electron microscope operating at 80 kV.

For CD, 60 µL sample was placed in a 0.1 mm quartz cuvette and placed into a JASCO spectropolarimeter. 100 µL ThS in 20 mM MOPS buffer was added to 50 µL of each sample to a final concentration of 20 µM, mixed well, incubated at room temperature for 10 minutes then fluorescence intensity measured in a Cary Eclipse spectrophotometer using an excitation wavelength of 440 nm. Baseline readings from 10 mM PB were subtracted from CD and ThS measurements.

The findings from these three experiments indicate that s1D12 inhibits the assembly of dGAE into fibrils in vitro.

Firstly, dGAE fibrils were readily observed by TEM in samples using concentrations as low as 10 µM; at the same concentration in the presence of s1D12, no fibrils were present suggesting inhibition of assembly. Fibrils were present when using the same molar ratios of dGAE:Ab with a non-tau antibody, showing that the inhibitory effect is specific.

Circular dichroism reports on secondary structure characteristics of proteins in solution. Although 10 and 25 µM dGAE were too low in concentration to be observed using CD, and the signal is dominated by the characteristic β-sheet signal expected from the antibody structure. At 100 µM dGAE, a random coil confirmation is revealed when the antibody CD signal is subtracted from the dGAE signal. This further indicates that for s1D12:dGAE at a ratio of 4:1, dGAE cannot assemble into β-sheet rich fibrils.

Finally, Thioflavin S was used to report on the presence of fibrils. ThS is a dye that binds to amyloid, the underlying structure in dGAE fibrils, and fluoresces at a characteristic wavelength of around 483 nm when excited with light of a wavelength of 440 nm. A positive signal was clearly observed for 25 and 100 µM dGAE fibrils. However, when incubated with s1D12 at ratios of either 4:1 or 1:1, this signal was abolished, supporting the previous observations that no fibrils are present when dGAE is incubated with s1D12.

Collectively, these results provide evidence that (i) s1D12 specifically inhibits the assembly of dGAE into fibrils and (ii) these assays can be utilized to determine the inhibitory activity of other antibodies in disrupting formation of fibrils that strongly resemble the paired helical filaments that are present in the tau pathology characteristic of Alzheimer's disease.

## Claims

1. An in vitro method of screening for an agent effective in inhibiting cytotoxicity of a fragment of Tau protein comprising at least 70 amino acids in the amino acid sequence of dGAE95 (SEQ ID NO:4) or a sequence with at least 85% identity thereto to a neuronal cell comprising the steps of:
(a) culturing the neuronal cell in the presence of the agent and subsequently culturing the neuronal cell with the fragment of Tau protein in the absence of heparin; or
(b) culturing the neuronal cell with the fragment of Tau protein in the absence of heparin and subsequently culturing the neuronal cell in the presence of the agent; and
(c) determining the cytotoxicity of the fragment of Tau protein to the neuronal cell after performing step (a) or step (b).

2. An in vitro method of screening for an agent effective in inhibiting internalisation of a fragment of Tau protein comprising at least 70 amino acids in the amino acid sequence of dGAE95 (SEQ ID NO:4) or a sequence with at least 85% identity thereto comprising the steps of:
(a) culturing the neuronal cell in the presence of the agent and subsequently culturing the neuronal cell with the fragment of Tau protein in the absence of heparin; or;
(b) culturing the neuronal cell of with the fragment of Tau protein in the absence of heparin and subsequently culturing the neuronal cell in the presence of the agent; and
(c) determining the internalisation of the fragment of Tau protein in the neuronal cell after performing step (a) or step (b).

3. An in vitro method of screening for an agent effective in disrupting interaction of a fragment of Tau protein comprising at least 70 amino acids in the amino acid sequence of dGAE95 (SEQ ID NO:4) or a sequence with at least 85% identity thereto with endogenous tau protein comprising the steps of:
(a) culturing the neuronal cell in the presence of the agent and subsequently culturing the neuronal cell with the fragment of Tau protein in the absence of heparin; or;
(b) culturing the neuronal cell with the fragment of Tau protein in the absence of heparin and subsequently culturing the neuronal cell in the presence of the agent; and
(c) determining the extent of interaction of the fragment of Tau protein with endogenous tau protein in the neuronal cell after performing step (a) or step (b).

4. A method as claimed in any one of claims 1 to 3, wherein the fragments of Tau protein as defined above for use according to the invention may be from 70 to 97 amino acids in length, optionally from 71 to 97 amino acids in length.

5. A method as claimed in claim 4, wherein the fragment of Tau protein is selected from the group of fragments of 71, 73, 94, 95, 97 amino acids in length.

6. A method as claimed in any one of claims 1 to 3, wherein the fragment of Tau protein comprising at least 70 amino acids in the amino acid sequence of dGAE95 (SEQ ID NO:4) or a sequence with at least 85% identity thereto is selected from the group consisting of fragments of Tau protein having amino acid sequences as described in SEQ ID NO:4, SEQ ID NO: 3, SEQ ID NO: 5, SEQ ID NO: 6, or SEQ ID NO: 7, or a sequence with at least 85% identity thereto.

7. A system for screening for an agent effective in inhibiting cytotoxicity of a fragment of Tau protein comprising at least 70 amino acids in the amino acid sequence of dGAE95 (SEQ ID NO:4) or a sequence with at least 85% identity thereto comprising (i) a neuronal cell line (ii) a fragment of Tau protein comprising at least 70 amino acids in the amino acid sequence of dGAE95 (SEQ ID NO:4) or a sequence with at least 85% identity thereto; and (iii) a library of agents; wherein the neuronal cell line is free from heparin, and wherein the agents are chemical compounds or specific binding molecules which can alter the effect of aggregates of tau protein, or a fragment thereof, on a neuronal cell.

8. A system for screening for an agent effective in inhibiting internalisation of a fragment of Tau protein comprising at least 70 amino acids in the amino acid sequence of dGAE95 (SEQ ID NO:4) or a sequence with at least 85% identity thereto comprising (i) a neuronal cell line. (ii) a fragment of Tau protein comprising at least 70 amino acids in the amino acid sequence of dGAE95 (SEQ ID NO:4) or a sequence with at least 85% identity thereto; and (iii) a library of agents; wherein the neuronal cell line is free from heparin, and wherein the agents are chemical compounds or specific binding molecules which can alter the effect of aggregates of tau protein, or a fragment thereof, on a neuronal cell.

9. A system for screening for an agent effective in disrupting interaction of a fragment of Tau protein comprising at least 70 amino acids in the amino acid sequence of dGAE95 (SEQ ID NO:4) or a sequence with at least 85% identity thereto with endogenous tau protein comprising (i) a neuronal cell line. (ii) a fragment of Tau protein comprising at least 70 amino acids in the amino acid sequence of dGAE95 (SEQ ID NO:4) or a sequence with at least 85% identity thereto; and (iii) a library of agents; wherein the neuronal cell line is free from heparin, and wherein agents are chemical compounds or specific binding molecules which can alter the effect of aggregates of tau protein, or a fragment thereof, on a neuronal cell.

10. A system as claimed in any one of claims 7 to 9, wherein the fragments of Tau protein as defined above for use according to the invention may be from 70 to 97 amino acids in length, optionally from 71 to 97 amino acids in length.

11. A system as claimed in claim 10, wherein the fragment of Tau protein is selected from the group of fragments of 71, 73, 94, 95, 97 amino acids in length.

12. A system as claimed in any one of claims 7 to 9, wherein the fragment of Tau protein comprising at least 70 amino acids in the amino acid sequence of dGAE95 (SEQ ID NO:4) or a sequence with at least 85% identity thereto is selected from the group consisting of fragments of Tau protein having amino acid sequences as described in SEQ ID NO:4, SEQ ID NO: 3, SEQ ID NO: 5, SEQ ID NO: 6, or SEQ ID NO: 7, or a sequence with at least 85% identity thereto.

## Patentansprüche

1. In-vitro-Verfahren zum Screening nach einem Wirkstoff, der die Zytotoxizität eines Fragments von Tau-Protein, das wenigstens 70 Aminosäuren in der Aminosäuresequenz von dGAE95 (SEQ ID NO:4) oder einer damit zu wenigstens 85 % identischen Sequenz umfasst, gegenüber einer Nervenzelle wirksam hemmt, umfassend die Schritte:
(a) Kultivieren der Nervenzelle in Gegenwart des Wirkstoffs und danach Kultivieren der Nervenzelle mit dem Fragment von Tau-Protein in Abwesenheit von Heparin; oder
(b) Kultivieren der Nervenzelle mit dem Fragment von Tau-Protein in Abwesenheit von Heparin und danach Kultivieren der Nervenzelle in Gegenwart des Wirkstoffs; und
(c) Bestimmen der Zytotoxizität des Fragments von Tau-Protein gegenüber der Nervenzelle nach Durchführen von Schritt (a) oder Schritt (b).

2. In-vitro-Verfahren zum Screening nach einem Wirkstoff, der die Internalisierung eines Fragments von Tau-Protein, das wenigstens 70 Aminosäuren in der Aminosäuresequenz von dGAE95 (SEQ ID NO:4) oder einer damit zu wenigstens 85 % identischen Sequenz umfasst, wirksam hemmt, umfassend die Schritte:
(a) Kultivieren der Nervenzelle in Gegenwart des Wirkstoffs und danach Kultivieren der Nervenzelle mit dem Fragment von Tau-Protein in Abwesenheit von Heparin; oder;
(b) Kultivieren der Nervenzelle von mit dem Fragment von Tau-Protein in Abwesenheit von Heparin und danach Kultivieren der Nervenzelle in Gegenwart des Wirkstoffs; und
(c) Bestimmen der Internalisierung des Fragments von Tau-Protein in der Nervenzelle nach Durchführen von Schritt (a) oder Schritt (b).

3. In-vitro-Verfahren zum Screening nach einem Wirkstoff, der die Wechselwirkung eines Fragments von Tau-Protein, das wenigstens 70 Aminosäuren in der Aminosäuresequenz von dGAE95 (SEQ ID NO:4) oder einer damit zu wenigstens 85 % identischen Sequenz umfasst, mit endogenem tau-Protein wirksam stört, umfassend die Schritte:
(a) Kultivieren der Nervenzelle in Gegenwart des Wirkstoffs und danach Kultivieren der Nervenzelle mit dem Fragment von Tau-Protein in Abwesenheit von Heparin; oder;
(b) Kultivieren der Nervenzelle mit dem Fragment von Tau-Protein in Abwesenheit von Heparin und danach Kultivieren der Nervenzelle in Gegenwart des Wirkstoffs; und
(c) Bestimmen des Ausmaßes der Wechselwirkung des Fragments von Tau-Protein mit endogenem tau-Protein in der Nervenzelle nach Durchführen von Schritt (a) oder Schritt (b).

4. Verfahren nach einem der Ansprüche 1 bis 3, wobei die Fragmente von Tau-Protein mit der oben angegebenen Bedeutung zur Verwendung gemäß der Erfindung eine Länge von 70 bis 97 Aminosäuren, gegebenenfalls von 71 bis 97 Aminosäuren aufweisen können.

5. Verfahren nach Anspruch 4, wobei das Fragment von Tau-Protein aus der Gruppe von Fragmenten mit einer Länge von 71, 73, 94, 95, 97 Aminosäuren ausgewählt ist.

6. Verfahren nach einem der Ansprüche 1 bis 3, wobei das Fragment von Tau-Protein, das wenigstens 70 Aminosäuren in der Aminosäuresequenz von dGAE95 (SEQ ID NO:4) oder einer damit zu wenigstens 85 % identischen Sequenz umfasst, aus der Gruppe bestehend aus Fragmenten von Tau-Protein mit Aminosäuresequenzen gemäß SEQ ID NO:4, SEQ ID NO: 3, SEQ ID NO: 5, SEQ ID NO: 6 oder SEQ ID NO: 7 oder einer damit zu wenigstens 85 % identischen Sequenz ausgewählt ist.

7. System für Screening nach einem Wirkstoff, der die Zytotoxizität eines Fragments von Tau-Protein, das wenigstens 70 Aminosäuren in der Aminosäuresequenz von dGAE95 (SEQ ID NO:4) oder einer damit zu wenigstens 85 % identischen Sequenz umfasst, wirksam hemmt, umfassend (i) eine Nervenzelllinie, (ii) ein Fragment von Tau-Protein, das wenigstens 70 Aminosäuren in der Aminosäuresequenz von dGAE95 (SEQ ID NO:4) oder einer damit zu wenigstens 85 % identischen Sequenz umfasst; und (iii) eine Bibliothek von Wirkstoffen; wobei die Nervenzelllinie frei von Heparin ist und wobei es sich bei den Wirkstoffen um chemische Verbindungen oder spezifisch bindende Moleküle handelt, die die Wirkung von Aggregaten von tau-Protein oder einem Fragment davon auf eine Nervenzelle verändern können.

8. System für Screening nach einem Wirkstoff, der die Internalisierung eines Fragments von Tau-Protein, das wenigstens 70 Aminosäuren in der Aminosäuresequenz von dGAE95 (SEQ ID NO:4) oder einer damit zu wenigstens 85 % identischen Sequenz umfasst, wirksam hemmt, umfassend (i) eine Nervenzelllinie, (ii) ein Fragment von Tau-Protein, das wenigstens 70 Aminosäuren in der Aminosäuresequenz von dGAE95 (SEQ ID NO:4) oder einer damit zu wenigstens 85 % identischen Sequenz umfasst; und (iii) eine Bibliothek von Wirkstoffen; wobei die Nervenzelllinie frei von Heparin ist und wobei es sich bei den Wirkstoffen um chemische Verbindungen oder spezifisch bindende Moleküle handelt, die die Wirkung von Aggregaten von tau-Protein oder einem Fragment davon auf eine Nervenzelle verändern können.

9. System für Screening nach einem Wirkstoff, der die Wechselwirkung eines Fragments von Tau-Protein, das wenigstens 70 Aminosäuren in der Aminosäuresequenz von dGAE95 (SEQ ID NO:4) oder einer damit zu wenigstens 85 % identischen Sequenz umfasst, mit endogenem tau-Protein wirksam stört, umfassend (i) eine Nervenzelllinie, (ii) ein Fragment von Tau-Protein, das wenigstens 70 Aminosäuren in der Aminosäuresequenz von dGAE95 (SEQ ID NO:4) oder einer damit zu wenigstens 85 % identischen Sequenz umfasst; und (iii) eine Bibliothek von Wirkstoffen; wobei die Nervenzelllinie frei von Heparin ist und wobei es sich bei Wirkstoffen um chemische Verbindungen oder spezifisch bindende Moleküle handelt, die die Wirkung von Aggregaten von tau-Protein oder einem Fragment davon auf eine Nervenzelle verändern können.

10. System nach einem der Ansprüche 7 bis 9, wobei die Fragmente von Tau-Protein mit der oben angegebenen Bedeutung zur Verwendung gemäß der Erfindung eine Länge von 70 bis 97 Aminosäuren, gegebenenfalls von 71 bis 97 Aminosäuren aufweisen können.

11. System nach Anspruch 10, wobei das Fragment von Tau-Protein aus der Gruppe von Fragmenten mit einer Länge von 71, 73, 94, 95, 97 Aminosäuren ausgewählt ist.

12. System nach einem der Ansprüche 7 bis 9, wobei das Fragment von Tau-Protein, das wenigstens 70 Aminosäuren in der Aminosäuresequenz von dGAE95 (SEQ ID NO:4) oder einer damit zu wenigstens 85 % identischen Sequenz umfasst, aus der Gruppe bestehend aus Fragmenten von Tau-Protein mit Aminosäuresequenzen gemäß SEQ ID NO:4, SEQ ID NO: 3, SEQ ID NO: 5, SEQ ID NO: 6 oder SEQ ID NO: 7 oder einer damit zu wenigstens 85 % identischen Sequenz ausgewählt ist.

## Revendications

1. Procédé *in vitro* de criblage pour un agent efficace dans l'inhibition de la cytotoxicité d'un fragment de protéine Tau comprenant au moins 70 acides aminés dans la séquence d'acides aminés de dGAE95 (SEQ ID NO: 4) ou une séquence comportant au moins 85 % d'identité avec celle-ci envers une cellule neuronale comprenant les étapes de :
(a) culture de la cellule neuronale en la présence de l'agent et subséquemment culture de la cellule neuronale avec le fragment de protéine Tau en l'absence d'héparine ; ou
(b) culture de la cellule neuronale avec le fragment de protéine Tau en l'absence d'héparine et subséquemment culture de la cellule neuronale en la présence de l'agent ; et
(c) détermination de la cytotoxicité du fragment de protéine Tau envers la cellule neuronale après la réalisation de l'étape (a) ou de l'étape (b).

2. Procédé *in vitro* de criblage pour un agent efficace dans l'inhibition de l'internalisation d'un fragment de protéine Tau comprenant au moins 70 acides aminés dans la séquence d'acides aminés de dGAE95 (SEQ ID NO: 4) ou une séquence comportant au moins 85 % d'identité avec celle-ci comprenant les étapes de :
(a) culture de la cellule neuronale en la présence de l'agent et subséquemment culture de la cellule neuronale avec le fragment de protéine Tau en l'absence d'héparine ; ou ;
(b) culture de la cellule neuronale avec le fragment de protéine Tau en l'absence d'héparine et subséquemment culture de la cellule neuronale en la présence de l'agent ; et
(c) détermination de l'internalisation du fragment de protéine Tau dans la cellule neuronale après la réalisation de l'étape (a) ou de l'étape (b).

3. Procédé *in vitro* de criblage pour un agent efficace dans la perturbation de l'interaction d'un fragment de protéine Tau comprenant au moins 70 acides aminés dans la séquence d'acides aminés de dGAE95 (SEQ ID NO: 4) ou une séquence comportant au moins 85 % d'identité avec celle-ci avec une protéine Tau endogène comprenant les étapes de :
(a) culture de la cellule neuronale en la présence de l'agent et subséquemment culture de la cellule neuronale avec le fragment de protéine Tau en l'absence d'héparine ; ou ;
(b) culture de la cellule neuronale avec le fragment de protéine Tau en l'absence d'héparine et subséquemment culture de la cellule neuronale en la présence de l'agent ; et
(c) détermination de l'étendue de l'interaction du fragment de protéine Tau avec la protéine Tau endogène dans la cellule neuronale après la réalisation de l'étape (a) ou de l'étape (b).

4. Procédé selon l'une quelconque des revendications 1 à 3, les fragments de protéine Tau étant comme définis ci-dessus pour une utilisation selon l'invention pouvant être de 70 à 97 acides aminés de longueur, éventuellement de 71 à 97 acides aminés de longueur.

5. Procédé selon la revendication 4, le fragment de protéine Tau étant choisi dans le groupe de fragments de 71, 73, 94, 95, 97 acides aminés de longueur.

6. Procédé selon l'une quelconque des revendications 1 à 3, le fragment de protéine Tau comprenant au moins 70 acides aminés dans la séquence d'acides aminés de dGAE95 (SEQ ID NO: 4) ou une séquence comportant au moins 85 % d'identité avec celle-ci étant choisi dans le groupe constitué par des fragments de protéine Tau ayant des séquences d'acides aminés telles que décrites dans la SEQ ID NO: 4, la SEQ ID NO: 3, la SEQ ID NO: 5, la SEQ ID NO: 6, ou la SEQ ID NO: 7, ou une séquence comportant au moins 85 % d'identité avec celle-ci.

7. Système pour le criblage pour un agent efficace dans l'inhibition de la cytotoxicité d'un fragment de protéine Tau comprenant au moins 70 acides aminés dans la séquence d'acides aminés de dGAE95 (SEQ ID NO: 4) ou une séquence comportant au moins 85 % d'identité avec celle-ci comprenant (i) une lignée de cellules neuronales (ii) un fragment de protéine Tau comprenant au moins 70 acides aminés dans la séquence d'acides aminés de dGAE95 (SEQ ID NO: 4) ou une séquence comportant au moins 85 % d'identité avec celle-ci ; et (iii) une bibliothèque d'agents ; la lignée de cellules neuronales étant exempte d'héparine, et les agents étant des composés chimiques ou des molécules de liaison spécifique qui peuvent modifier l'effet d'agrégats de protéine Tau, ou d'un fragment correspondant, sur une cellule neuronale.

8. Système pour le criblage pour un agent efficace dans l'inhibition de l'internalisation d'un fragment de protéine Tau comprenant au moins 70 acides aminés dans la séquence d'acides aminés de dGAE95 (SEQ ID NO: 4) ou une séquence comportant au moins 85 % d'identité avec celle-ci comprenant (i) une lignée de cellules neuronales, (ii) un fragment de protéine Tau comprenant au moins 70 acides aminés dans la séquence d'acides aminés de dGAE95 (SEQ ID NO: 4) ou une séquence comportant au moins 85 % d'identité avec celle-ci ; et (iii) une bibliothèque d'agents ; la lignée de cellules neuronales étant exempte d'héparine, et les agents étant des composés chimiques ou des molécules de liaison spécifique qui peuvent modifier l'effet d'agrégats de protéine Tau, ou d'un fragment correspondant, sur une cellule neuronale.

9. Système pour le criblage pour un agent efficace dans la perturbation de l'interaction d'un fragment de protéine Tau comprenant au moins 70 acides aminés dans la séquence d'acides aminés de dGAE95 (SEQ ID NO: 4) ou une séquence comportant au moins 85 % d'identité avec celle-ci avec une protéine Tau endogène comprenant (i) une lignée de cellules neuronales, (ii) un fragment de protéine Tau comprenant au moins 70 acides aminés dans la séquence d'acides aminés de dGAE95 (SEQ ID NO: 4) ou une séquence comportant au moins 85 % d'identité avec celle-ci ; et (iii) une bibliothèque d'agents ; la lignée de cellules neuronales étant exempte d'héparine, et des agents étant des composés chimiques ou des molécules de liaison spécifique qui peuvent modifier l'effet d'agrégats de protéine Tau, ou d'un fragment correspondant, sur une cellule neuronale.

10. Système selon l'une quelconque des revendications 7 à 9, les fragments de protéine Tau étant comme définis ci-dessus pour une utilisation selon l'invention pouvant être de 70 à 97 acides aminés de longueur, éventuellement de 71 à 97 acides aminés de longueur.

11. Système selon la revendication 10, le fragment de protéine Tau étant choisi dans le groupe de fragments de 71, 73, 94, 95, 97 acides aminés de longueur.

12. Système selon l'une quelconque des revendications 7 à 9, le fragment de protéine Tau comprenant au moins 70 acides aminés dans la séquence d'acides aminés de dGAE95 (SEQ ID NO: 4) ou une séquence comportant au moins 85 % d'identité avec celle-ci étant choisi dans le groupe constitué par des fragments de protéine Tau ayant des séquences d'acides aminés telles que décrites dans la SEQ ID NO: 4, la SEQ ID NO: 3, la SEQ ID NO: 5, la SEQ ID NO: 6, ou la SEQ ID NO: 7, ou une séquence comportant au moins 85 % d'identité avec celle-ci.
